# EUROPEAN PATENT SPECIFICATION

(11) **EP 2 734 187 B1**
(45) Date of publication and mention of the grant of the patent: **05.09.2018**
(21) Application number: 12762404.7
(22) Date of filing: 19.07.2012
(51) Int. Cl.: A61K 9/06, A61P 13/00, A61K 9/00, A61K 47/10, A61K 47/34, A61K 47/38

(54) **MATERIALS AND METHOD FOR TREATING INTERNAL BODY CAVITIES**
MATERIALEN UND VERFAHREN ZUR BEHANDLUNG INNERER KÖRPERHOHLRÄUME
MATÉRIAUX ET MÉTHODE DE TRAITEMENT DE CAVITÉS CORPORELLES INTERNES

(30) Priority: 20.07.2011 US 201161509654 P
(43) Date of publication of application: 28.05.2014
(73) Proprietor: UroGen Pharma Ltd., Ra'anana 4365007 (IL)
(72) Inventor: DE LA ZERDA, Jaime, 34751 Haifa (IL); DOLLBERG, Yosh, 43362 Raanana (IL); SHPOLANSKY. Uri, Karkur, 37038 Israel (IL); HAKIM, Gil, 43596 Ra'anana. (IL); KONORTY, Marina, 46446 Hertzlya (IL)
(74) Representative: Pohlman, Sandra M.
(86) International application number: PCT/IL2012/000284
(87) International publication number: WO 2013/011504

(56) References cited:
- EP-A2- 0 386 960
- WO-A2-00/50004
- WO-A2-2011/089604
- US-A1- 2004 009 212
- US-A1- 2005 266 086
- HE C ET AL: "In situ gelling stimuli-sensitive block copolymer hydrogels for drug delivery", JOURNAL OF CONTROLLED RELEASE, ELSEVIER, AMSTERDAM, NL, vol. 127, no. 3, 8 May 2008 (2008-05-08), pages 189-207, XP022617735, ISSN: 0168-3659, DOI: 10.1016/J.JCONREL.2008.01.005 [retrieved on 2008-01-26]

## Description

### REFERENCE TO RELATED APPLICATION

This application claims priority from U.S. Provisional Pat. Appl. No. 61/509,654, filed 20 July 2011.

### FIELD OF THE INVENTION

The invention relates in general to materials and means for sustained release of therapeutic agents for topical treatments. It relates in particular to means for topical treatment of diseases of internal body cavities by embedding therapeutic agents in a slowly degrading biocompatible mixture applied to affected tissue, being the residues of said mixture expelled by the natural flow of bodily fluids. The material compositions are based upon general common properties of bodily organs and optimized for each particular application depending on the type of tissue and organ to be treated.

### BACKGROUND OF THE INVENTION

### i. Internal cavities

Internal body cavities (e.g., cavities within the body accessible through a body orifice or via a minimal invasive techniques such as image guided laparoscopic methods) include organs that when diseased may benefit from prolonged direct exposure to certain drugs. These cavities are naturally wet and are either continuously flushed or generate a flow of body fluids, such as urine or serous fluids - that contribute to gradual expelling of treatment materials.

The main benefit of topical treatment is that drugs effect is mostly contained to tissue in the immediate vicinity of the targeted delivery - in contrast to drugs that are delivered and distributed through the blood system and can potentially adversely affect other organs and tissues.

The main challenge is specific and accurate delivery of drug into cavities and maintaining it essentially in contact with the targeted tissue throughout internal organ movements.

Many cavities are characterized by their internal natural movement - due, for example, to body motion or peristaltic motion - that constantly changes their shape.

Tissue in all internal cavities is either epithelial, connective or muscular. Epithelial cells rest on connective tissue, which provides nutrition and binds to neighboring structures. The epithelia of vessels and serous cavities are squamous, but many other cavities have epithelia structure that provides mechanical support and ability to achieve adhesion. The mouth, esophagus, larynx, vagina and anal canal have a non-keratinized cell structure to maintain wetness and are more challenging for adhesion; the urinary tract has transitional cell structure, that provides stretching, mechanical strength and strongest resistance to adhesion of materials.

Several common tissue properties affect the requirements for targeted delivery materials:
- wetness of the cavity lining tissue
- mechanical composition and consistency
- absorption capability
- expansion and movement characteristics of the internal cavities or attached organs
- existence of body fluids for expelling the delivery materials

### ii. Internal cavity membranes

### Mucous membranes

Mucous membranes are the moist linings of the orifices and internal parts of the body that are in continuity with the external surface. All membranes have a surface layer of epithelial cells over a deeper layer of connective tissue and contain mucus-secreting cells scattered among other cells.

The lining layers which are closest to the transition from the skin - in the mouth, anus, and vagina -are layers of thin cells. Most other sites have a single layer of tall 'columnar' cells, flat 'squamous' cells, or intermediate 'cuboidal' cells.

In the urinary tract, the mucous membranes that line the urethra, bladder, and ureters are several cells thick, allowing, especially in the bladder, for expansion; the particular protection required here is against the acidity of the urine. (These linings are in continuity with that of the kidney pelvisand in turn the ducts and tubules leading to the thin glomeruli membranes, which filter the blood.)

### Serous membranes

A serous membrane is a smooth membrane consisting of a thin layer of cells which secrete serous lubricating fluid which reduces friction from muscle movement.

The pleura is a serous membrane which folds back onto itself to form a two-layered, membrane structure. The thin space between the two pleural layers is known as the pleural cavity; it normally contains a small amount of pleural fluid. The outer pleura is attached to the chest wall -the inner covers the lungs.

The peritoneum is the serous membrane that forms the lining of the abdominal cavity supporting the abdominal organs. The outer layer is attached to the abdominal wall The inner is wrapped around the organs located inside the intraperitoneal cavity. The potential space between these two layers is the peritoneal cavity; it is filled with of serous fluid that allows the two layers to slide freely over each other.

### Physiological properties of internal cavity tissues

Distinct types of epithelial cells and mucus-secreting cells cover the internal surfaces of the human body. The mucosal surfaces serve many vital functions, such as respiration (nasal passage and lung), absorption (gastrointestinal tract), excretion (lung, urinary tract, large intestine), and reproduction (reproductive tract).

### iii. Topical treatment of diseases

Targeted delivery can focus the therapeutic effect onto a target organ, providing therapeutic benefits and minimizing side effects. Some drugs have an optimum concentration range within which maximum benefit is derived, and concentrations above or below this range can be toxic or produce non-optimal therapeutic benefit . Sustained release of a drug involves compositions formulated to release a drug at a controlled rate by means of diffusion out of the composition or by dilution of the composition over time or both.

Topical administration of drugs can optimize the therapeutic effect by controlling the drug release rate and their adsorption by the tissue. Water-based gel compositions are the optimal delivery mechanism and present the additional characteristic of adhesiveness to the treated tissue, which allows for a prolonged contact between the composition and the organ. Examples of drugs applied in the form of gels include the anti-inflammatory voltaren and the anticonvulsant diazepam ("Diastat").

### iv. Topically administered drugs

Some drugs that are administered topically belong to the following families:
1. Antineoplastic drugs
2. Chemotherapeutic agents
3. Anti-infective agents (e.g. antimicrobial drugs, antiparasitic agents, antivirals)
4. Genito-urinary system drugs
5. Anti-inflammatory drugs
6. Analgesics
7. Musculoskeletal system acting drugs
8. Drugs acting on the blood and blood forming organs (antihemorrhagics, antithrombotic agents, antianemic drugs)
9. Dermatologic drugs (antifungals, antiseptic)
10. Gastrointestinal system (antiobesity, acid-related disorders)
11. Metabolism drugs
12. Neurological drugs
13. Respiratory drugs including nasal drugs
14. Cardio-vascular drugs
15. Otological drugs
16. Corticosteroids drugs
17. Analgesics drugs
18. Antiparasitics drugs
19. Anesthetic drugs

In some cases, the topical treatment is just evolving:
20. Growth factor (e.g., for treatment of heart muscle ischemia)
21. Gene therapy agents

In other cases, the topically administered material has medical effect without actually being considered an active pharmaceutical ingredient:
22. Mucin
23. Hyaluronic Acid

Besides their therapeutic effect, drugs are classified according to their chemical and physical properties and thus constitute families of compounds that, due to their common similar characteristics, fit particular drug vehicles. Some of those properties include:
- Lipophilicity or hydrophilicity
- Molecular weight and physical size
- Diffusability through different media which is hydrophobic, hydrophilic, viscose, etc.
- Solubility

### v. Physical characteristics of internal cavities

The effectiveness of topical application of a therapeutic agent to a specific internal cavity depends on the physical characteristics of the internal cavity tissue, in particular, characteristics such as:
- Access - ease of introducing liquid or gel into the cavity
- Tissue adhesiveness - ability to attach gel to the cavity tissue
- Internal movement- effected by gravitational motion, stretching, peristaltic motion, etc. that cause periodical changes in the shape and volume of the cavity
- Wetness - that enables diffusion of drug into tissue
- Degradability mechanism - flow of liquids e.g. urine, or serous fluids
- Mechanics of flow of materials inside the organ. For example: Urine in the bladder, which accumulates, then is released at once; menstrual fluids in the vagina; bolus in the intestine, which advance in virtue of the peristaltic movementetc..
- Acidity -The extreme acidity of the stomach, for example, is a different medium than the slightly basic intestine medium or slightly acid medium in the urinary tract. The medium acidity may be utilized to trigger the release of drugs.

The specific cavity characteristics require careful consideration in the development of materials for topical treatment of diseases. An example is the enhancement of drug absorption by bladder tissue which is lined withmucin that constitutes a barrier to be penetrated before the tissue is engaged. The matrix composition optimally includes components that modify the mucin to ease penetration of the active pharmaceutical ingredient into the affected tissue.

A summary of some internal organs, types of tissue their inner lining is made of, and characteristics of the surrounding medium is presented in Table 1.

**Table 1**

| System | Organ | Type of Tissue | Elasticity / Mobility | pH | Surroudings |
|---|---|---|---|---|---|
| Digestive | Mouth/Tongue | Mucosal | Very | Neutral | Flow of food and liquids |
| | Esophagus | Mucosal | None | Neutral | Flow of food and liquids |
| | Stomach | Mucosal | Very | Strongly acidic | Very acidic fluids, enzimatic activity |
| | Duodenum | Mucosal | Moderate | Slightly alakine | Flow of bolus, enzymatic activity |
| | Small intestine | Mucosal, ciliated | Moderate | Slightly alakine | Flow of bolus, enzymatic activity, absorption of nutrients |
| | Large intestine | Mucosal | Moderate | Slightly alakine | Flow of feces |
| | Rectum | Mucosal | Moderate | Slightly alakine | Flow of feces |
| Respiratory | Mouth/Nose | Mucosal | None | Neutral | Flow of air and humidity |
| | Larinx | Mucosal | Moderate | Neutral | Flow of air and humidity |
| | Eustachian tubes | Mucosal, ciliated | None | Neutral | Presence of air, humidity |
| | Pharinx | Mucosal | Moderate | Neutral | Flow of air and humidity |
| | Thrachea | Mucosal | None | Neutral | Flow of air and humidity |
| | Lungs/Alveoli | Mucosal | Moderate | Neutral | Flow of air and humidity |
| Urinary | Kidneys | Mucosal | None | Slightly acidic | Blood filtration, production of urine |
| | Ureters | Mucosal | None | Slightly acidic | Flow of urine |
| | Bladder | Mucosal | Very | Slightly acidic | Accumulation and evacuation of urine |
| | Prostate | Mucosal | None | Slightly acidic | Flow of urine and semen (slightly alkaline in ejaculation) |
| | Urethra | Mucosal | Moderate | Slightly acidic | Flow of urine and semen (in the males) |
| Genital | Vas deferens | Mucosal, ciliated | None | Neutral | Flow of seminal fluids |
| | Epididymis | Mucosal, ciliated | None | Neutral | Flow of seminal fluids |
| | Testes | Mucosal | Moderate | Neutral | Production of seminal fluids Coitus, flow of semen, flow of menstrual |
| | Vagina | Mucosal | None | Neutral | fluids |
| | Cervix | Mucosal | Moderate | Neutral | Flow of semen, flow of menstrual fluids |
| | Fallopian tubes | Mucosal, ciliated | None | Neutral | Flow of semen, flow of menstrual fluids |
| | Ovules | Mucosal | None | Neutral | Production of ova |
| Pleura | Pleural membranes | Serous | Moderate | Neutral | Pleural fluids |
| Peritoneum | Peritoneal membranes | Serous | Moderate | Neutral | Peritoneal fluids |

### vi. Chemotherapy - anticancer drugs

In the particular case of treatment of TCC cancer in the urinary tract, the bladder tissue penetration by chemotherapy drugs - a critical parameter in treatment effectiveness - exhibits a linear relationship with the concentration of the chemotherapy drugs (see Gao X, Au JL, Badalament RA, Wientjes MG. Bladder tissue uptake of mitomycin C during intravesical therapy is linear with drug concentration in urine. Clin Cancer Res. 1998 Jan;4(1):139-43)). Furthermore, chemotherapy drug penetration is 40% higher in the tumor tissue than in the adjacent normal urothelium. Gao et al demonstrated double Mitomycin C (MMC) concentration in tissue when instilling 40mg/20ml compared with 20 mg/20ml MMC: human bladder tumors have a significantly higher tissue MMC uptake than regular bladder tissue.

The anti-tumor effect of chemotherapy drugs depends on concentration and exposure time. Schmittgen et al (see Schmittgen TD, Wientjes MG, Badalament RA, Au JL. Pharmacodynamics of mitomycin C in cultured human bladder tumors. Cancer Res. 1991 Aug 1;51(15):3849-56) demonstrated, both in TCC cell cultures and human bladder tumor tissue cultures, that a ten times higher concentration was needed in order to get a similar cell kill effect when exposure time to MMC was reduced from 24 hours to two hours.

The proven conclusion is that maintaining higher drug concentration for longer treatment duration will enhance the treatment efficacy. Furthermore, sustained delivery of chemotherapy enables the use of lower drug concentration - thus reducing the risk of adverse events - and still get superior therapeutic effect.

### vii. Anti-inflammatory drugs

Anti-inflammatory drugs make up about half of analgesics, curing pain by reducing inflammation. They include steroids or corticosteroids and non-steroidal that differ in nature and mode of action. Among the most well-known non-steroidal anti-inflammatory drugs, are aspirin, ibuprofen and naproxen.

Adverse reactions are dose-dependent and mainly gastrointestinal (ulcer perforation, upper gastrointestinal bleeding and dyspepsia) - estimated to result in 103,000 hospitalizations and 16,500 deaths per year in the United States.

Bladder cystitis can be treated by direct deposition of gel composition containing the drug (e.g, anti-inflammatory drug like ibuprofen) on the internal wall of the bladder - thus avoiding systemic drug uptake and the potential hazardous adverse effects.

### viii. Required properties for topical treatment in the urinary tract and other internal cavities

The of material compositions used for topical administration of therapeutic agents for treatment of diseases in internal body cavities such as the urinary tract must be designed to the needed properties that deliver the required medical effect. Important properties include:
- Rheological properties (viscosity, thixotropy, gelling temperature - gel point) - required for the convenient introduction of the material into the internal cavity
- Adhesion - required to coat dependably the target tissue
- Flexibility - to comply with the natural volume and shape changes of the internal cavity under treatment
- Dilution in aqueous media - to enable API release and natural expelling of the material through body fluids. In the case of hydrophobic drugs, additives such as pharma-approved surface-active agents may be required to enhance the drug's dissolution or dispersion in aqueous media
- Mechanical properties, such as hardness, tensile strength to provide the required resilience, depending on the organ. For example, if there is a continuous flow of material or fluid in the organ, the matrix/mixture requires additional robustness so that it may not be dragged
- Duration of time that the material remains in the internal cavity before it degrades
- A suitable therapeutic agent, containing Active Pharmaceutical Ingredient (API) and excipients -or drug derivative chosen from the families listed in section iii.
- Loading of drug or API in the material. For certain clinical protocol the amount and concentration of the drug or API mixed into the material have to be set to a prescribed level. The amount of therapeutic agent thus used can be significantly lower than used in regular parallel instillations and more than a single API can be loaded. So the API part of the administered material may vary from zero concentration (gel only) to 50% (e.g., for DMSO).
- The ability of the matrix/mixture to release the drug in a controlled manner such that the actual drug concentration vis-à-vis the organ tissue or lining upon which the mixture is adhered will be optimal for each treatment. It is precisely the specific composition of the mixture that determines the release profile of the drug and its adsorption into the target tissue. For example, if the API is lipophilic, the addition of certain surface-active agents in given concentrations will provide for their emulsification, easier release from the drug composition and easier absorption by the internal organ lining.
- Drug viability. The material is designed and tested not to reduce the viability duration of the drug or API that is mixed into it, so that the amount that is released throughout the treatment will have the optimal therapeutic effect.

Limitations of Superficial Bladder Cancer (SBC) treatments known in the art: SBC is a highly-recurrent form of cancer. To lower recurrence, it is considered necessary to treat patients with a single intravesical chemotherapy instillation immediately after trans-Uretral Resection of Tumors (TUR-T).

A meta-analysis of 7 randomized trials (1,476 patients with a median follow-up of 3.4 years) has demonstrated that one chemotherapy instillation immediately after TUR-T decreases the relative risk of recurrence by 40% (see Sylvester RJ, Oosterlinck W, van der Meijden AP. A single immediate postoperative instillation of chemotherapy decreases the risk of recurrence in patients with stage Ta T1 bladder cancer: a meta-analysis of published results of randomized clinical trials. J Urol. 2004 Jun;171(6 Pt 1):2186-90). The timing of the instillation is crucial: in all studies, instillation was administered within 24 hours. A study reported that if the first instillation was not given within 24 hours, the risk of recurrence increased twofold (see Kaasinen E, Rintala E, Hellström P, Viitanen J, Juusela H, Rajala P, Korhonen H, Liukkonen T; FinnBladder Group. Factors explaining recurrence in patients undergoing chemoimmunotherapy regimens for frequently recurring superficial bladder carcinoma. Eur Urol. 2002 Aug; 42(2):167-74).

According to EAU guidelines, following resection patients are stratified by their risk for tumor progression/recurrence:
- Patients with low risk (30%) - need no further instillations.
- Intermediate risk patients (40-50%) - usually receive 6 additional sessions of Mitomycin C (MMC) chemotherapy instillations.
- High risk patients (20%) - are usually treated with 6 Bacillus Calmette-Guerin (BCG) intravesical instillations.

The efficacy of chemotherapy intravesical instillations for Superficial Bladder Cancer is limited, because there is no control on the chemotherapy amount and the duration until it is expelled. In an attempt to prolong the standard treatment to two hours, some physicians dictate behavioral conditions to reduce acidity of the bladder, to reduce urine volume before instillation and instill maximal concentration of chemotherapy dissolved in a volume of saline.

There are several known obstacles that presently complicate the coating of the internal wall of the bladder (and hence for topical treatments for bladder cancer):
- The mucosal membrane. A physiological purpose of the mucosal membrane that covers the bladder wall is to prevent adherence of foreign bodies to it. Therefore, a composition targeted to adhere to the internal wall of the bladder has to overcome this difficulty. Furthermore, it is not acceptable in present medical practice to dry the mucosal layer that is soaked in urine before coating it. Another complication stems from the delicate membrane structure of 'umbrella' cells that are the urothelium most superficial layer. Regular bio-adhesives, such that are used to stop bleeding (e.g. Tabotamp from Johnson & Johnson), bond strongly through wet surfaces and peel-off that delicate, outermost layer and thus damage the membrane. Achieving a satisfactory non-damaging coating of wet, non-adherent mucosal tissue is very challenging. Furthermore, a drug carrier composition should ideally modify the mucin layer to allow better drug penetration to the urothelium.
- The bladder's natural expansion and collapse. The bladder wall is naturally highly flexible. Bladder volume varies from collapsed or 'empty' state of 0-30 ml up to a filled bladder volume of up to 600 ml (though the micturition point is usually 150-200 ml). Therefore, a composition that can adhere and conform to the bladder wall without damaging the outer layer, adapt itself to the bladder's morphology and stay adhered throughout volume changes , while avoiding the peeling-off of the outermost layer of the membrane is considered an enormous challenge.

- When access to an internal body cavity is required, an applicator such as a flexible endoscope or catheter is utilized. In the context of the present invention, the applicator allows the injection of the therapeutic gel into the internal organ and its direct application upon the inner lining of the organ.
- The same obstacles, to even greater extent, are relevant to the treatment of the same cancer (transitional cell carcinoma) in the upper urinary tract. TCC in the upper urinary tract is a rare urological disease and has a propensity for multifocality, local recurrence, and development of metastases. Almost 5% of all urothelial neoplasms occur in the kidney and ureters. The standard treatment for patients with upper tract TCC and a normal kidney is a complete removal of the involved kidney, ureter and bladder cuff. A less-invasive treatment, namely resection of tumors followed by instillation with chemotherapy or immunotherapy, is recommended for patients with anatomic or functional solitary kidneys, bilateral upper-tract TCC, base line renal insufficiency, or inability to tolerate major surgery. Patients with a normal contralateral kidney who have small, low-grade lesions can also be reasonable candidates for this organ conserving management.

Topical immunotherapy or chemotherapy instillations for treatment of UTUC are used as primary or adjuvant treatment in order to reduce tumor recurrence. Topical instillation is performed using either infusion through a percutaneous nephrostomy tube, via a retrograde ureteral catheter, or by retrograde reflux from the bladder with an indwelling double-J stent. The main disadvantage in all these treatments is the short residual duration of the active agent in the treated area resulting in a low exposure time essential for treatment efficacy. This may be one of the reasons for the shorter average disease-free duration of upper tract TCC patients, compared to lower tract TCC patients.
- Another bladder disease is overactive bladder (OAB) - when the bladder contracts suddenly without patient's control when the bladder is not full. This syndrome affects an estimated 1 in 11 adults in the United States - especially common in older adults.

Current available treatment options for OAB are: bladder training, pelvic floor exercises, drug therapy such as anti-cholinergics, capsaicin, intravesical botulinum toxin injections and in severe cases- bladder augmentation surgery.

Current oral drugs have high adverse event rate which leads to patients' intolerability. Bladder instillation with antimuscarines has been tried with lower adverse event rate, but require recurrent catheterization due to the drug relatively short half- life that reduces compliance.

Despite promising results the drawbacks to intravesical botulinum toxin injections are numerous: the cystoscopic injection requires proficiency and authorization of the physician, some degree of anesthetic administration is required, the botulinum toxin effects only the injected anatomical locations and the treatment may lead to temporary urinary retention and need for self -catheterization.

Common to the examples above is the need to reach the inner wall of an internal cavity whereby the access to said internal cavity is difficult.

### ix. Mechanical support and sustained drug release in minimally-invasive surgery

In laparoscopic surgery, the surgeon needs to achieve adequate retraction and exposure and stabilize "moving targets" while operating on non-fixed organs. Current solutions include adding more access ports or using a hand-assisted technique or the use of temporary sutures through the abdominal wall. A solution in which the organs can be held mechanically in place without the need for manual assistance is thus a long-felt need.

### x. Current state-of-the-art

To the best of the inventors' knowledge, a method for treating diseases of the bladder or other inner cavities such as other urinary tract organs, reproductive organs, respiratory organs, alimentary organs and non-open organs like the pleura and the peritoneum based on production of a solidified coating layer and affixing it onto the internal wall of the organ cavity, followed by continuous release of the therapeutic agent(s) from the coating, remains unknown in the art.

Furthermore, the application of the substrate material such that it creates a continuous layer substantially affixed to the mucosal lining of the outermost tissue of the internal cavity for prolonging the exposure of the drug to the targeted cells is neither trivial nor obvious to any person skilled in the art.

Specifically, compositions known in the prior art as sustained-release substrates for the treatment of bladder cancer (for example, the invention disclosed in U.S. Pat. Appl. US2006/0127420 to Chung) are lipophilic (oil-based). Given that the inner bladder wall is mucosal, essentially and permanently soaked in an aqueous medium (i.e., urine), a drug embedded in a hydrophilic medium would more effectively diffuse through the matrix/mixture and conveniently reach the bladder wall, allowing in that way an intimate, continuous contact between the drug and the bladder wall.

Thus, there remains a long-felt and unmet need for a material with the following properties: it is hydrophilic; it provides a homogeneous layer that can securely adhere to the surfaces of internal body cavities, in particular, mucosal tissue of such cavities as the bladder; it remains attached despite the natural motions of the tissue to which it is attached; it is easily applied; it assists in overcoming the mucosal barrier and allowing the drug to reach the actual organ tissue; it is biocompatible; it provides a continuous sustained release of a therapeutic agent; the rate of release of the therapeutic agent is determined by the concentration of the agent and the rate of degradation of the material; and as the material degrades, it is excreted from the body by the body's own natural processes.

Additional properties that are required for the treatment of other internal cavities, include additional capabilities such as the ability to connect between tissues by adhesive forces and the ability to prevent connection between contacting tissues.

To the best of the inventors' knowledge, a method to design a material with the specific properties that are required to provide optimal treatment in any internal cavity, as exemplified by the treatment of bladder diseases remains unknown in the art.

### SUMMARY OF THE INVENTION

The invention disclosed herein is designed to meet this long-felt need. The use of a thermoreversible hydrogel in a system for delivery of a therapeutic agent is disclosed. Because the hydrogel has a much lower viscosity at low temperatures (about 10 °C) than at body temperature, it can be inserted into a body cavity at low temperature as a liquid and will then gel against the body tissue as it warms to room temperature.

It is therefore an object of the present invention to disclose the use of a thermoreversible hydrogel composition in a system for delivery of a therapeutic agent to the urinary tract, characterized in that said thermoreversible hydrogel composition comprises a thermoreversible hydrogel characterized by: a viscosity of less than 200 Pa·s over a temperature range of 4 °C - 12 °C; a viscosity of greater than 10³ Pa·s at 37 °C; a "peel strength" adhesiveness according to ASTM standard D2256-03 of 0.5 - 5.0 N cm⁻² at 37 °C; and, flexibility sufficient such that a 3 cm² × 3 cm² section of bladder tissue layered with said thermoreversible hydrogel at room temperature can be stretched to 9 cm² × 9 cm² without detachment of said thermoreversible hydrogel from said bladder tissue.

It is a further object of this invention to disclose such a use of a thermoreversible hydrogel composition in a system for delivery of a therapeutic agent to the urinary tract, wherein said composition additionally comprises an effective amount of at least one therapeutic agent for treatment of the urinary tract.

It is a further object of this invention to disclose the use of a hydrogel composition in a system for delivery of a therapeutic agent to the pleura, characterized in that said hydrogel composition comprises a thermoreversible hydrogel characterized by a viscosity of less than 5 Pa·s over a temperature range of 4 °C - 12 °C; a viscosity of greater than 10³ Pa·s at 37 °C; a "peel strength" adhesiveness according to ASTM standard D2256-03 of 0.5 - 5.0 N cm⁻² at 37 °C; and flexibility sufficient such that a 3 cm² × 3 cm² section of bladder tissue layered with said thermoreversible hydrogel at room temperature can be stretched to 9 cm² × 9 cm² without detachment of said thermoreversible hydrogel from said bladder tissue.

It is a further object of this invention to disclose the use of a hydrogel composition in a system for delivery of a therapeutic agent to the pleura, wherein said composition additionally comprises an effective amount of a therapeutic agent for treatment of the pleura.

It is a further object of this invention to disclose the use of a thermoreversible hydrogel composition in a system for delivery of a therapeutic agent to a mucosal or serous membrane, characterized in that said hydrogel composition comprises a thermoreversible hydrogel characterized by a viscosity of less than 5 Pa·s over a temperature range of 4 °C - 12 °C; a viscosity of greater than 10³ Pa·s at 37 °C; a "peel strength" adhesiveness according to ASTM standard D2256-03 of 0.5 - 5.0 N cm⁻² at 37 °C; and flexibility sufficient such that a 3 cm² × 3 cm² section of bladder tissue layered with said thermoreversible hydrogel at room temperature can be stretched to 9 cm² × 9 cm² without detachment of said thermoreversible hydrogel from said bladder tissue.

It is a further object of this invention to disclose such a use of a thermoreversible hydrogel composition in a system for delivery of a therapeutic agent to a mucosal or serous membrane, wherein said composition additionally comprises an effective amount of a therapeutic agent for treatment of a mucosal or serous membrane.

It is a further object of this invention to disclose such a use of a thermoreversible hydrogel composition in a system for delivery of a therapeutic agent to a mucosal or serous membrane, wherein said mucosal or serous membrane is located at an internal cavity chosen from the group consisting of , mouth, nasal sinus, paranasal sinus, gallbladder, esophagus, rectum, lungs, vagina, uterus, stomach, renal pelvis, pleura, abdomen, peritoneum, pelvis, liver, kidney, heart, intestine, brain, and vertebral column.

In some embodiments of the invention, said thermoreversible hydrogel is further characterized by a viscosity of less than 5 Pa·s over a temperature range of 4 °C - 12 °C. In some embodiments of the invention, the said thermoreversible hydrogel is further characterized by a viscosity of less than 0.5 Pa·s over a temperature range of 4 °C - 12 °C. In some embodiments of the invention, the thermoreversible hydrogel is further characterized by a viscosity of greater than 3 × 10³ at 37 °C.

In some embodiments of the invention, said thermoreversible hydrogel has a "peel strength" adhesiveness according to ASTM standard D2256-03 of 1.0 - 5.0 N cm⁻² at 37 °C.

In some embodiments of the invention, said thermoreversible hydrogel is additionally characterized by flexibility sufficient to permit the volume of the tissue to which said composition is applied to expand by a factor of at least 3 without detachment of said gel from said tissue.

In some embodiments of the invention, said thermoreversible hydrogel comprises between 20% and 30% (w/w) ethylene oxide/propylene oxide block copolymer; between 0.05% and 0.5% (w/w) hydroxypropylmethylcellulose (HPMC); between 0.1% and 2.5% (w/w) polyethylene glycol (PEG)-400; and the balance water.

In some embodiments of the invention, said thermoreversible hydrogel comprises between 20% and 30% (w/w) ethylene oxide/propylene oxide block copolymer; between 0.1% and 0.3% (w/w) HPMC; between 0.1% and 1.8% (w/w) PEG-400; and the balance water.

In some embodiments of the invention, said thermoreversible hydrogel comprises between 18% and 40% (w/w) ethylene oxide/propylene oxide block copolymer; between 0.05% and 0.8% (w/w) CMC; between 0.1% and 2.5% (w/w) PEG-400; and the balance water.

In some embodiments of the invention, said thermoreversible hydrogel comprises between 12-30% Pluronic F127; between 5-30% Pluronic F68; between 0.05% and 2% (w/w) CMC; between 0.1% and 2.5% (w/w) PEG-400; and the balance water.

It is a further object of this invention to disclose such a use as defined in any of the above, wherein said thermoreversible hydrogel additionally comprises at least one component selected from the group consisting of adhesive and thickening compounds; bonding agents; pH-modifying substances; diffusion coatings; plasticizers; components for increasing permeability within the formulation; swellable excipients; matrix forming polymers; tight junction modifiers / cell membrane permeability enhancers; and any combination thereof.

In some embodiments of the invention, said bonding agent is selected from the group consisting of polycarbophil, cellulose, microcrystalline cellulose, cellulose derivatives, HPMC, hydroxypropylcellulose (HPC), low-substituted hydroxypropylcellulose, dicalcium phosphate, lactose, sucrose, ethylcellulose, hydroxypropymethylcellulose acetate succinate (HPMCAS), polyvinylpyrrolidone (PVP), vinylpyrrolidone/vinyl acetate copolymer, polyethylene glycol, polyethylene oxide, polymethacrylates, polyvinyl alcohols (PVA), partially hydrolysed polyvinyl acetate (PVAc), polysaccharides, hyaluronic acid, fats, fatty acid derivatives, and any combination thereof.

In some embodiments of the invention, said diffusion coating is chosen from the group consisting of ethylcelluloses and polymethacrylates, cellulose acetate and cellulose acetate butyrate or any combination thereof.

In some embodiments of the invention, said component for increasing permeability within the formulation is chosen from the group consisting of polyethylene glycols, PVP, PVA, HPMC, HPC, hydroxyethylcelluloses (HEC), methylcellulose (MC), carboxymethylcelluloses and their salts, dextrins, maltodextrins, cylcodextrins, dextrans, urea, salts, sugars, sugar alcohols, and any combination thereof.

In some embodiments of the invention, said swellable excipient is selected from the group consisting of polyvinylpyrrolidones, crospovidones, crosslinked sodium carboxymethylcellulose, crosslinked sodium carboxymethylstarch, polyethylene oxides, polymethyacrylates, L-HPC, cellulose acetate, ethylcellulose, polymethacrylates, high-molecular weight polyethylene oxides, xanthan gum, copolymers of vinylpyrrolidone and vinyl acetate, polyvinylpyrrolidones, crospovidones, crosslinked sodium carboxymethylcellulose, crosslinked sodium carboxymethylstarch, poly(hydroxyalkyl methacrylate), alginates, galactomannans, and any combination thereof.

In some embodiments of the invention, said matrix forming polymer is selected from the group consisting of hydroxyethylmethylcelluloses, HPC, HEC, MC, ethylcelluloses, alkylcelluloses, hydroxyalkylcelluloses, hydroxyalkylmethylcelluloses, sodium CMCs, alginates, galactomannans, xanthans, polyethylene oxides, polyacrylic acids, polymethacrylic acids, polymethacrylic acid derivatives, polyvinyl alcohols, partially hydrolysed polyvinyl acetate, polyvinylpyrrolidone, agar, pectin, gum arabic, tragacanth, gelatin, starch, starch derivatives poly(propylene oxide) (PPO), poly(lactide-co-glycolic acid) (PLGA), poly(N-isopropylacrylamide) (PNIPAM), poly(propylene fumarate) (PPF), polyurethane (PU), poly(organophosphazene) (POP), stearic acid, poly(acrylic acid), glyceryl stearate, cetearyl alcohol, sodium stearoyl lactylate, hydroxy-lanolin, and any combination thereof.

In some embodiments of the invention, said tight junction modifier / cell membrane permeability enhancer is chosen from the group consisting of anionic surfactants, non-anionic surfactants, charged polymers, dimethyl sulfoxide (DMSO), decylmethyl sulfoxide, tert-butyl cyclohexanol, fatty acids their esters and salts, ethanol, nicotinamide, urea, perfluoropolyether, monoterpene ketones, disodium citrate, succinic acid and tris.

In some embodiments of the invention, said surfactant is chosen from the group consisting of polysorbates, sodium dodecyl sulfate, and dextran sulfate.

In some embodiments of the invention, said charged polymer is chosen from the group consisting of chitosan poly-arginine, polylysine, and aliginate.

In some embodiments of the invention, said monoterpene ketone is chosen from the group consisting of like (-) menthol, (-) menthone, peppermint oil, and spearmint oil.

In some embodiments of the invention, said thermoreversible hydrogel is additionally used as a biological glue.

It is a further object of this invention to disclose such a use as defined in any of the above, further characterized in that said composition releases said therapeutic agent, over a temperature range of 36 °C - 42 °C and a pH range of between 5.5 and 8.0, at a rate of 80% in a time range of between 3 and 30 hours.

It is a further object of this invention to disclose such a use as defined in any of the above, further characterized in that said composition releases said therapeutic agent, over a temperature range of 36 °C - 42 °C and a pH range of between 1 and 9.0, at a rate of 80% in a time range of between 3 and 30 hours.

It is a further object of this invention to disclose such a use as defined in any of the above, further characterized in that said composition releases said therapeutic agent, over a temperature range of 36 °C - 42 °C and a pH range of between 1 and 9.0, at a rate of 80% in a time range of between 2 and 4 weeks.

It is a further object of this invention to disclose such a use as defined in any of the above, wherein said therapeutic agent for treatment of the urinary tract is selected from the group consisting of antineoplastic agents, chemotherapeutic agents, anti-infective agents, antimicrobial agents, antiparasitic agents, antiviral agents, agents acting on the blood, antihemorrhagics, antithrombotic agents, antifungals, antiseptics, agents for treating diseases of the genito-urinary system, anti-inflammatory agents, neurological agents, gene therapy agents, corticosteroids, analgesic and anesthetic agents, growth factors, VEGF, inhibitory factors, LIF, proteins, mucin, and any combination thereof.

It is a further object of this invention to disclose such a use as defined in any of the above, wherein said therapeutic agent for treatment of the urinary tract is selected from the group consisting of Mitomycin C, Deoxrubicin, Valrubicin, and Gemcitabine, Thiotepa, Ethoglucid (Epodyl), Epirubicin, Pirarubicin, Apaziquone,Vicinium and botulinium toxin, and interleukin-2.

It is a further object of this invention to disclose such a use as defined in any of the above, wherein said system further comprises a second hydrogel composition used subsequently, said second hydrogel composition comprising a thermoreversible hydrogel characterized by a viscosity of less than 200 Pa·s over a temperature range of 4 °C - 12 °C; a viscosity of greater than 10³ Pa·s at 37 °C; a "peel strength" adhesiveness according to ASTM standard D2256-03 of 0.5 - 5.0 N cm⁻² at 37 °C; and flexibility sufficient such that a 3 cm² × 3 cm² section of bladder tissue layered with said thermoreversible hydrogel at room temperature can be stretched to 9 cm² × 9 cm² without detachment of said thermoreversible hydrogel from said bladder tissue; and an effective amount of a cell cycle modifying agent for synchronization of cell cycle chosen from the group consisting of chalones, colcemid, methotrexate, and thymidine.

It is a further object of this invention to disclose such a use as defined in any of the above, wherein said system is further characterized in that it is designed to release said therapeutic agent continuously for at least 12 hours.

### BRIEF DESCRIPTION OF THE FIGURES

The invention disclosed herein is described with reference to the figures, in which
FIG. **1** presents the flow diagram of the creation of a hydrogel composition in a drug delivery system for treatment of internal cavities;
FIG. **2** presents graphs illustrating the MMC cytotoxicity when different MMC concentrations are used in saline and in DTC-2 respectively;
FIG. **3** Study of the effect of botulinum toxin gel on contraction of animal bladder as model for overactive bladder contraction. A. contraction amplitude B. inter-contraction interval;
FIG. **4** presents semi-logarithmic graph of viscosity as a function of temperature for different embodiments of reverse thermal hydrogels;
FIG. **5** presents in vitro release of Lidocaine hydrochloride (10 and 20 mg/ml) from reverse thermal hydrogel (including 27% Ethylene Oxide/Propylene Oxide Block Copolymer, 1% PEG-400, 0.2% Hydroxypropylmethyl cellulose in water) and Cephalexin (1mg/ml) from reverse thermal hydrogel (20.0% Pluronic F-127, 10% Pluronic F-68 (10.0%) 1.0% PEG-400 0.4% CMC sodium in water; and,
FIG. **6** presents various dissolution rates of six hydrogel compositions.

### DETAILED DESCRIPTION OF THE PREFERRED EMBODIMENTS

For explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figure and described in the specification, but only as indicated in the accompanying claims, with the proper scope determined only by the broadest interpretation of said claims.

The current invention comprises a series of systems that combine therapeutic materials and application means for the topical treatment of diseases that are focused in internal cavities, such as a system for treating Superficial Bladder Cancer (SBC), or a system for treating pleural effusion and other forms of diseases - inflammatory, infectious and other.

The material composition of the present invention not only allows direct deposition but also the controlled release of drug upon the diesease area for optimal therapeutic effect.

The present invention provides a design of such systems that is based on the characteristics of the internal cavities to be treated and the specific requirements for said treatments in order to determine the required properties of hydrogel systems that can satisfy all these requirements.

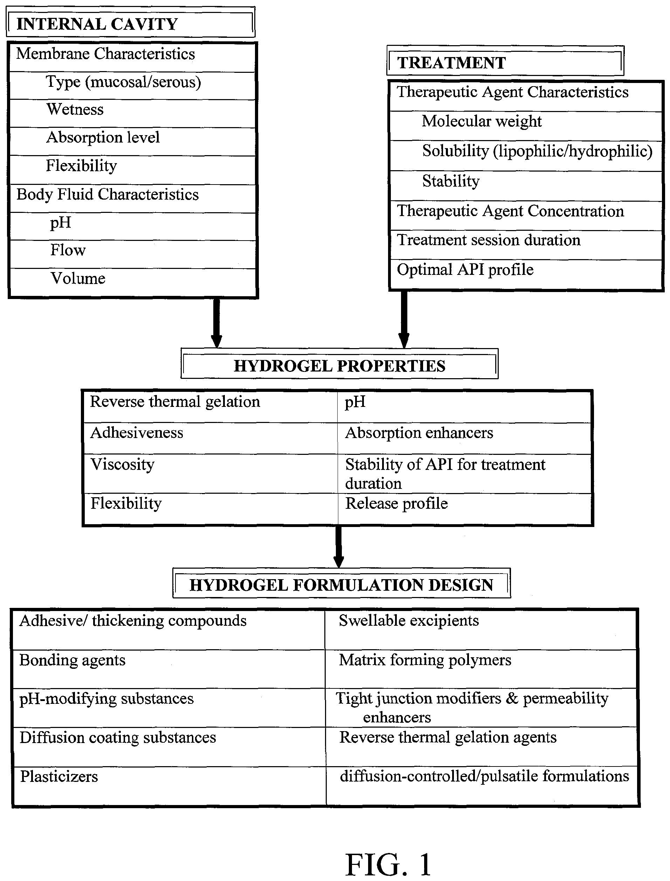

The present invention provides a structured design and test flow that assures optimal hydrogels for optimal treatments. This structured set of hydrogel properties enables the formulation of the hydrogel systems for optimal drug delivery. A flow diagram for creation of hydrogel composition is illustrated in FIG. **1****.**

The present invention provides a bioerodible, biocompatible gel mixed with an active agent, such as chemotherapy agent like MMC or gemcitabine, or immunotherapy such as BCG (bacillus Calmette-Guerin), which is inserted into the upper urinary tract via a catheter, solidifies and forms a drug reservoir at the destined treatment area such as the renal pelvis.

The diffusion of the drug from the gel and the erosion of the gel by the urine or the biodegradability of the gel, deliver drug to the tissue - producing prolonged high topical drug concentration but low systemic exposure. This, the system increases bioavailability, reduces toxicity and improves treatment efficacy. Furthermore, loading the material with significant concentration of chemotherapy agent and applying the material directly over a tumor for a prolonged duration, may ablate non-resected tumors that are in close contact with the material.

The present invention provides a bioerodible, biocompatible hydrogel that that is mixed with a neurological drug such as botulinum toxin, is inserted into the bladder, solidifies and forms a drug reservoir inside the bladder. The erosion of the material in the urine, promotes topical drug delivery and increases the drug affinity to the bladder wall tissue producing high topical drug concentration in the bladder wall and at the same time keeps a low systemic exposure. The system provides drug delivery to the entire bladder resulting in total tissue contact with the delivered neurological drug as opposed to delivery induced by local injections.

The invented material includes a mixture of hydrogel with botulinium toxin (A, B, Cl, D, E, F and G and equivalent neurological drugs) for the treatment of disorders characterized by bladder spasms (eg. urge incontinence due to unstable bladder or unstable detrusor, sphincter or neurogenic bladder, etc.). The hydrogel mixed with botulinium toxin will be instilled into the bladder without injections into the bladder tissue and its effect will be.

These systems contain therapeutic agents embedded in a slowly dissolving biocompatible hydrogel and are combined with administering means, so that the materials can be introduced in a minimally invasive manner into a body cavity and provide a prolonged exposure of the cavity tissue to the drug, thus improving the treatment efficacy in terms of improved therapeutic effect of the drug and reduced tissue damage. The admix/mixture is biocompatible and dissolves in body fluids such as urine, serous fluids or lymphatic fluids, and then it is expelled from the body.

The hydrogels disclosed herein can be administered comfortably through a thin tube, for example a urethral catheter, exerting pressure similar to that required to inject saline through such a device; as the liquid hydrogel settles upon the bladder wall, it solidifies creating a gelatinous film that then gradually releases the therapeutic ingredient upon said bladder wall. Similarly, other drug-containing materials can be injected inside other internal cavities in the body and perform their therapeutic activity as they coat the corresponding inner lining of the organ and the drug is gradually released from them.

This feature is of fundamental importance and is enabled by the fact that the polymeric composition is designed so as to possess reverse thermal gelling properties, that is, to possess low viscosity at low temperatures (say, below 15 °C) and dramatically higher viscosity at body temperature.

The current invention thus also provides means by which the organs can be held mechanically in place during laparascopic surgery by injecting the invented materials into the cavity to solidify and support the internal organs. The invented materials and means have the additional advantages of a) serving as a soothing dressing for the surgery cuts, b) contributing to healing through sustained release of anti-infection drug and analgesic drug for a therapeutically-significant duration (e.g., over 6 hours), and c) avoiding the need for further surgical or medical procedure, by natural degradation of the material and its expelling from the body.

Similar methods, but with a different family of materials can be used to prevent the adhesion of tissues between organs in the treated area, which may often occur during laparoscopic surgery.

In one preferred embodiment, a urinary catheter is used that incorporates a large balloon with a volume of 70 cm³ to 140 cm³. The cold (and hence low-viscosity) material is intoduced into the bladder after the bladder has been essentially emptied to minimize the amount of urine in the hydrogel material. The balloon is inflated to open the essentially collapsed bladder into an essentially symmetrical shape that is not less than a quarter of the volume of the bladder when it is filled with urine and reaches the volume that may cause urge to void (micturition).

In a preferred embodiment, the catheter system includes means to measure the momentary pressure that the balloon is applying on the bladder wall as indication for the physician to determine the level of inflating of the balloon. As a non-limited example, such means can measure the pressure of the liquid inside the balloon by connecting the supply tube into the balloon to a transparent column and reading the change in heights of liquid in that column as indication to the change in pressure on the bladder wall induced by inflating the balloon.

In a preferred embodiment, the position of the patient is changed for each dose so that each dose is injected separately between the inflated balloon and downward toward the bladder wall - to utilize gravitation to assist material flow.

In a preferred embodiment means are provided to apply pressure on the material during its heating and gelation to help enhance its fixation and adhesion to the mucosal tissue of the bladder wall. In one preferred embodiment, the pressure is applied by the large balloon that is filled with liquid such as water or saline. In another - the balloon can be filled with a heavier liquid such as saturated salt water (26%, with typical density of 1.2 g/ml), or saturated solution of glucose in water.

In another preferred embodiment, the balloon contains magnetic material embedded into its design; in another non-limiting example the catheter balloon can contain a metal rod. In yet another non-limiting example the balloon can be inflated with ferromagnetic liquid such as ferrofluids, made of finely divided magnetite (a form of iron oxide that is ferromagnetic), an oily carrier and a surfactant like the composition produced by FerroTec, Inc - see http://www.ferrotec.com/products/ferrofluid/. In this example, the pressure is applied by an external magnet that is pulled to the magnet inside the balloon. The external magnet can be a stationary magnet and the patient can be positioned against it corresponding to the exit hole that will be injected through. In another arrangement, the magnet can be rotated around the patient. In another embodiment, the procedure can be administered inside a system such as an MRI unit that enables rotating the magnetic field electronically.

In a preferred embodiment, the instillation of doses can be repeated with a similar or different set of materials to achieve a second layer of coating with additional properties, such as blocking layer that will reduce the diffusion of material into the bladder and slow the natural degradation of the material into the urine in the bladder. In a non-limiting example the similar materials can be the polymer ingredient of the DTC material such as a mixture of one or more of PEG-400, PEG-800 or Poloxamer with distilled water. In a nonlimiting example, this mixture can be less viscous than the DTC material used for the first layer, thus having a slower diffusion rate and providing a barrier against release of drug into the bladder volume.

Following the instillation, the coating will release active ingredients with medical effect on the bladder disease, such as cancer fighting drugs that affect bladder cancer tumors. In parallel that coating will naturally and gradually dilute into the bladder urine and be expelled during urination.

In case it may become needed to speed this process of gradual dilution by external actions, in a preferred embodiment, the coating can be removed promptly by cooling the coating below its gelation temperature, thus significantly lowering its viscosity and speeding its dilution into the bladder urine. In a non-limited example, the cooling can be affected by flushing the bladder with a flow of cold liquid such as water or saline through a regular catheter into the bladder. Water and Saline will also help to melt water-based DTCx gels. In another preferred embodiment, the flow of cold liquid can be applied via a special catheter, which will enable a thin or focused jet of liquid that will exert pressure on a small part of the coating. In a preferred embodiment, the direction of this jet may be changed such that it can be activated on several areas of the bladder coating sequentially.

In another preferred embodiment, speeding the removal of the coating can be achieved by applying a chemical agent such as a solvent that can assist in the dissolution of the coating. The chemical agent can be chosen according to the formulation of the DTCx. As a non-limiting example, ethyl or isopropyl alcohol and DMSO (dimethyl suloxide) can be used to dissolve coatings that include lipophilic materials.

The aim of the prolonged exposure of target tissue to drugs released from the coating is to enhance the efficacy of the drug in topical treatment of that target tissue, while reducing potential systemic adverse effects to other organs.

The homogeneous coating obtained in the invention disclosed herein, i.e., a solidified and unified layer that provides continuous sustained release of therapeutic agents upon the inner surface of an internal body cavity, as herein disclosed, has to the inventors' knowledge never been used clinically.

As used herein, the term "internal cavity" is used to describe parts in the body that are either accessible through an orifice - e.g., mouth, bladder, intestine, esophagus, rectum, lungs, vagina, stomach, renal pelvis, etc. - or by way of minimally invasive, surgery - e.g., pleura, abdomen, peritoneum, pelvis, etc. The definition includes artificially made or enlarged cavities in adipose tissues and fibrous capsules in internal organs such as the kidney, heart, intestine, etc. that are accessible by image guided laparoscopic techniques.

As used herein, the term "DTC" is used to refer generically to the materials disclosed in the present invention. The terms "DTCx" and "DTC-*n*" (where n is an integer) are used to refer to particular embodiments, either generically (DTCx) or specifically (e.g. DTC-2).

The current invention provides a platform for the redesign of drugs to make them suitable for topical administration. The inventors' studies demonstrated solubility and first order sustained release of Mytomicin C, Doxorubicin and Gemcitabin (groups 1 & 2), Abamectin (group 3), exogenous glycosaminoglycan (group 4), Naproxen (group 5), lidocaine and voltaren (group 6) and botulinum toxin (group 12)

Among the drugs that can be administered topically are drugs that belong to the following families:
1. Antineoplastic drugs
2. Chemotherapeutic agents
3. Anti-infective agents (e.g. Antimicrobial drugs, Antiparasitic agents, Antivirals)
4. Genito-urinary system drugs
5. Anti-inflammatory products
6. Analgesics
7. Musculoskeletal system acting drugs
8. Drugs acting on the blood and blood forming organs (Antihemorrhagics, Antithrombotic agents, antianemic drugs)
9. Dermatologic drugs (antifungals, antiseptic)
10. Gastrointestinal system (antiobesity, acid related disorders)
11. Metabolism drugs
12. Neurological drugs
13. Respiratory drugs including nasal drugs
14. Cardio-vascular drugs
15. Otological drugs
16. Anti-infective drugs
17. Corticosteroids drugs
18. Analgesics drugs
19. Antiparasitics drugs
20. Anasthetic Drugs
21. Growth factor (e.g., for treatment of heart muscle ischemia)
22. Gene Therapy agents
23. Mucin
24. Hyaluronic Acid

Drugs can be embedded as part of the invented materials as a single therapeutic agent or as a combination. As an example, a mixture containing exogenous glycosaminoglycan and Naproxen can be combined in a specific material for the treatment of interstitial cystitis for alleviating the inflammation symptoms and replacing the damaged mucosal lining of the urinary bladder cavity that is typical for this disease.

The use of the novel medicinal formulations with controlled release of active ingredient is expected to achieve substantially more constant drug levels and avoid the occurrence of level peaks, thus enhancing the therapeutic efficacy and reducing unwanted side effects.

As standard, chemotherapy drugs are administered at a maximal concentration level that is tolerable by patients. The present invention study results demonstrate that a further improvement in efficacy can be gained by increasing the exposure time to chemotherapy drugs. This is at the core of the present invention.

In addition, the use of such formulation allows reduction of the frequency of administration thus leads to improved patient's compliance.

According to a preferred embodiment of the present invention the formulation described above, is for example in the form of active ingredient-containing hydrogels. These diffusion-controlled systems may be completely diluted in the hydrogel formulation or can be multiparticulate, i.e. they may consist of a large number of coated cores such as, for example, of microencapsulated APIs, where appropriate together with conventional excipients and carriers, as defined below for example, is applied and subsequently coated with a diffusion coating which may comprise plasticizers and other excipients. The diffusion-controlled systems according to the invention may additionally consist of homogeneous active ingredient-containing cores which are produced for example by granulation, rotor granulation, fluidized bed agglomeration, tableting, wet extrusion or melt extrusion, where appropriate with spheronization, and are coated with a diffusion coating which may comprise plasticizers and other excipients.

According to one embodiment, the present invention may provide a combination of APIs, one or more diluted or suspended in the gel and one or more microencapsulated for slower release effect. For example, an anesthetic like lidocaine dissolved in the formulation for immediate anesthetic effect and encapsulated MMC for cancer treatment that is released after the bladder wall is insensitive and the patient can withstand the MMC therapy.

According to a preferred embodiment of the present invention the material / formulation/mixture described above, additionally comprises at least one ingredient selected from
(a) adhesive and thickening compounds;
(b) bonding agents;
(c) pH-modifying substances;
(d) diffusion coating;
(e) plasticizers;
(f) Tight junction modifiers & permeabiulity enhancers
(g) swellable excipients matrix-forming polymers;
(h) diffusion-controlled or pulsatile formulations;
(i) reverse thermal gelation agents.

The adhesive and thickening compounds preferably used in the production of coated neutral pellets (e.g. consisting of sucrose, microcrystalline cellulose, citric acid) are polycarbophil (polymer of acrylic acid crosslinked with divinyl glycol), hydroxypropylmethylcellulose (HPMC) at varios degrees of methoxy substitution and polyvinylpyrrolidone (PVP). It is likewise possible to employ other naturally, synthetic or partially synthetic polymers such as, for example methylcellulose (MC), hydroxy-propylcellulose (HPC), other hydroxyalkylcelluloses and hydroxyalkylmethylcelluloses, carboxy-methylcelluloses and salts thereof, polyacrylic acids, polymethacrylates, gelatin, starch or starch derivatives including starch phosphate esters at various degrees of substitution, as well as gums like guar gum, locust beam gum and xanthan gum and their derivatives.

The bonding agents employed for the production of active ingredient -containing microcapsules are for example polycarbophil, cellulose, microcrystalline cellulose, cellulose derivatives such as, for example, HMPC, HPC and low-substituted hydroxypropylcellulose (L-HPC), dicalcium phosphate, lactose, PVP and sucrose, ethylcellulose, hydroxypropymethylcellulose acetate succinate (HPMCAS), PVP, vinylpyrrolidone/vinyl acetate copolymer, polyethylene glycol, polyethylene oxide, polymethacrylates, polyvinyl alcohols (PVA), partially hydrolysed polyvinyl acetate (PVAc), polysaccharides (e.g. alginic acid, alginates, galactomannans) waxes, fats and fatty acid derivatives.

The pH-modifying substances such as, for example, acids, bases and buffer substances are incorporated into the active ingredient-containing core. Addition of these substances makes it possible to reduce markedly the pH-dependence of the release of the APIs. Examples of suitable excipients which modify the pH in the active ingredient-containing cores are: adipic acid, malic acid, L-arginine, ascorbic acid, aspartic acid, benzenesulphonic acid, benzoic acid, succinic acid, citric acid, ethanesulphonic acid, 2-hydroxyethanesulphonic acid, fumaric acid, gluconic acid, glucuronic acid, glutamic acid, potassium hydrogen tartrate, maleic acid, malonic acid, methanesulphonic acid, toluenesulphonic acid, trometamol, tartaric acid, potassium hydrogen tartrate and their respective salts, Phosphate salts, Tris, sodium and potassium salts are preferably employed.

Particularly suitable for producing the diffusion coatings are ethylcelluloses and polymethacrylates such as, for example, Eudragit.RTM. NE, Eudragit.RTM. RS and RL. However, other materials such as, for example, cellulose acetate and cellulose acetate butyrate can also be employed as film-forming diffusion-controlling polymers.

Examples of plasticizers used are citric acid derivatives (e.g. triethyl citrate, tributyl citrate, acetyl triethyl citrate), phthalic acid derivatives (e.g. dimethyl phthalate, diethyl phthalate, dibutyl phthalate), benzoic acid and benzoic esters, other aromatic carboxylic esters (e.g. trimellithic esters), aliphatic dicarboxylic esters (e.g. dialkyl adipates, sebacic esters, in particular diethyl sebacate, tartaric esters), glycerol monoacetate, glycerol diacetate or glycerol triacetate, polyols (e.g. glycerol, 1,2-propanediol, polyethylene glycol of varying chain length), fatty acids and derivatives (e.g. glycerol monostearates, acetylated fatty acid glycerides, castor oil and other natural oils, Miglyol) and fatty acid alcohols (e.g. cetyl alcohol, cetylstearyl alcohol).

The nature and amount of the plasticizer are chosen so that the above-defined release according to the invention and the necessary stability of the medicinal forms is achieved. The proportion of the plasticizer is from 0 to 50%, preferably 0 to 35%, particularly preferably 0 to 25% based on the mass of the hydrogel composition.

The release rate according to the invention is controlled by the gel composition. Certain components may increase the permeability of the formulation including water-soluble polymers such as, for example, polyethylene glycols, PVP, PVA, HPMC, HPC, hydroxyethylcelluloses (HEC), MC, carboxymethylcelluloses or their salts, dextrins, maltodextrins, cylcodextrins, dextrans or other soluble substances such as, for example, urea, salts (sodium chloride, potassium chloride, ammonium chloride, etc.), sugars (sucrose, lactose, glucose, fructose, maltose etc.) and sugar alcohols (mannitol, sorbitol, xylitol, lactitol, etc.). Based on the mass of the hydrogel, the amount of the water-soluble polymers ranges from 0 to 50%, preferably 0 to 35%, particularly preferably 0 to 20%, increasing permeability components may be employed.

A further aspect of the present invention are coated formulation which comprise one or more swellable excipients which, on penetration of liquid through the membrane, swell greatly and, through the swelling and volume expansion, cause the coating to split. The splitting of the coating makes it possible for the medicinal substance to be released from the admix/formulation/mixture, usually in pulsatile form. Swellable excipients which these formulations may comprise are, for example, polyvinylpyrrolidones, crospovidones, crosslinked sodium carboxymethylcellulose, crosslinked sodium carboxymethylstarch, polyethylene oxides, polymethyacrylates, low-substituted hydroxypropylmethylcellulose (L-HPC). Examples of suitable coating materials are cellulose acetate, ethylcellulose and polymethacrylates.

The described diffusion-controlled or pulsatile formulations can be employed directly and unmodified as medicinal form. However, they may also be further processed, where appropriate with addition of excipients, to the final formulation. In order to achieve a desired release profile it is also possible to combine different coated formulations in one medicinal form, and administration of an initial dose can take place for example by combination with rapid-release formulation particles, e.g. uncoated pellets, granules or powder.

In a further embodiment of the formulation containing the controlled release ingredient. These so-called formulation release the active ingredient by diffusion and/or erosion.

The mass ratio of active ingredient to the total mass of the formulation in these novel formulations is in the range from 1:1 to 1:10,000, preferably in the range from 1:2 to 1:1,000. formulation Preference is additionally given to medicinal preparations in the context of this invention which comprise water-soluble, hydrogel-forming polymers, these polymers having a nominal viscosity of at least 50 mPa·s, (measured as 2% strength aqueous solution at 20° C.).

A preferred family of candidates to be utilized as a basis for obtaining said hydrogel is group of tri-block copolymers designated as PEG-PPG-PEG (PEG = Polyethylene glycol and PPG = Polypropylene glycol) and called Poloxamers, that produce reverse thermal gelation compositions, i.e., with the characteristic that their viscosity increases with increasing temperature up to a point from which viscosity again decreases. In particular, Poloxamer 407 also known a PF-127, aqueous solutions of 20 to 30% w/w possesses a gelling temperature which is above 10 °C but below the human body temperature, i.e., 37 °C. This characteristic may confer the ability of a composition containing the compound to be injected or infused in liquid state into a bodily inner cavity at a low temperature and, afterwards, as the composition warms, it solidifies into a gel, thus stabilizing upon the wall of the inner body cavity.

PF-127 is more soluble in cold water than in hot water. At low temperatures in aqueous solutions, a hydration layer surrounds PF-127 molecules. When the temperature is raised, the hydrophilic chains of the copolymer become desolved as a result of the breakage of the hydrogen bonds that had been formed between the solvent and these chains. This phenomenon favors hydrophobic interactions among the polyoxypropylene domains and leads to gel formation. Because of the dehydration process, the hydroxyl groups become more accessible. A liquid micellar phase is stable at low temperatures but transforms into the cubic structure by increasing the temperature. At higher temperatures, a phase of hexagonal packed cylinders is formed, as shown in FIG. 1.

Reverse thermal gelation and low toxicity have been the basis of research into the use of PF-127 as a possible drug delivery system in humans.

Aqueous solutions of poloxamers are stable in the presence of acids, alkalis, and metal ions. Commonly used poloxamers include the 88 (F-68 grade), 237 (F-87 grade), 338 (F-108 grade) and 407 (F-127 grade) types, which are freely soluble in water. The "F" designation refers to the flake form of the product. PF-127 has a good solubilizing capacity, low toxicity and is, therefore, considered a good medium for drug delivery systems.

Furthermore, PF-127 has been reported to be the least toxic of commercially available copolymers.In addition, other materials that induce reverse thermal gelation are: Alginates, cellulose acetophatalate and carbopol.

Water-swellable matrix-forming polymers preferably employed are hydroxy-propylmethylcelluloses (HPMC) of different degres of methoxy substitution, hydroxyethylmethylcelluloses, hydroxypropylcelluloses (HPC), hydroxyethylcelluloses methylcelluloses (MC), ethylcelluloses, other alkylcelluloses, hydroxy-alkylcelluloses and hydroxyalkylmethylcelluloses, sodium carboxymethylcelluloses (NaCMC), alginates, galactomannans such as, for example, guar and carob flour, xanthans, polyethylene oxides, polyacrylic acids, polymethacrylic acids, polymethacrylic acid derivatives, polyvinyl alcohols (PVA), partially hydrolysed polyvinyl acetate (PVAc), polyvinylpyrrolidone (PVP), agar, pectin, gum arabic, tragacanth, locust beam gum, gelatin, starch or starch derivatives, in particular starch phosphate esters and mixtures of these substances.

In this connection, the formulation according to the invention should preferably comprise at least 0.1-2.0% of a hydroxypropylmethylcellulose type whose nominal viscosity (measured as 2% strength aqueous solution at 20° C.) is at least 50 mPa·s, HPMC types preferably used have a degree of substitution of methoxy groups of 16.5-30%, particularly preferably 19-30%, and a degree of substitution of hydroxypropoxy groups of 4-32%, particularly preferably 4-12%. CMC salts types preferably used have nominal viscosity (measured as 1% strength aqueous solution at 20° C.) is at least 15 mPa·s.formulation formulation.Examples of plasticizing excipients in the hydrogel formulation are propylene glycol, glycerol, triethylene glycol, butanediols, pentanols, such as pentaerythritol, hexanols, long-chain alcohols, polyethylene glycols, polypropylene glycols, polyethylene/propylene glycols, silicones, phthalic acid derivatives (e.g. dimethyl phthalate, diethyl phthalate, dibutyl phthalate), benzoic acid and benzoic esters, other aromatic carboxylic esters (e.g. trimellithic esters), citric acid derivatives (e.g. triethyl citrate, tributyl citrate, acetyl triethyl citrate), aliphatic dicarboxylic esters (e.g. dialkyl adipates, sebacic esters, in particular diethyl sebacate, tartaric esters), glycerol monoacetate, glycerol diacetate or glycerol triacetate, fatty acids and derivatives (e.g. glycerol monostearates, acetylated fatty acid glycerides, castor oil and other natural oils, Miglyol), fatty acid alcohols (e.g. cetyl alcohol, cetylstearyl alcohol), sugars, sugar alcohols and sugar derivatives (e.g. erythritol, isomalt, lactitol, mannitol, maltitol, maltodextrin, xylitol). The concentration of plasticizers is normally from 0 to 30%, preferably from 0 to 20% based on the total mass of the gel.

Examples of further suitable water-swellable polymers which may be incorporated in the hydrogel are high-molecular weight polyethylene oxides, xanthan gum, copolymers of vinylpyrrolidone and vinyl acetate, polyvinylpyrrolidones, crospovidones, crosslinked sodium carboxymethylcellulose, crosslinked sodium carboxymethylstarch, low-substituted hydroxypropylmethylcellulose (L-HPC), poly(hydroxyalkyl methacrylate), alginates and galactomannans and mixtures thereof.

The present invention further relates to the combination of formulations with different release properties, e.g. rapid-release and slow-release, in one medicinal form.

If necessary, the pH can be adjusted to 5.5-9.0 with a buffer composition. The release is carried out at a temperature of 36-42° C.

If necessary, the pH can be adjusted to 1-5.5 with a buffer composition. The release is carried out at a temperature of 36-42° C.

According to one embodiment of the present invention provides a hydrogel composition that is intended to be used as a topical therapeutic agent, applied topically. Thus, it would be useful to have a device that allows the application of such said hydrogel composition once accessing a body organ (inner cavity) through an orifice (either natural or artificial), so as to allow the coating of the inner wall of the organ with said hydrogel gel. Such hydrogel composition will adhere well to said wall and will (i) allow the controlled release of the drug or drugs; (b) will enable the gradual dilution of the hydrogel; and, (c) expelled from the body in a reasonable period of time (e.g., 24 hrs).

The average release rate in the context of the present invention is defined via the time until the release of active ingredient reaches 80%, whereas the initial release describes the percentage release of active ingredient after 30 minutes.

The formulation according to the invention with controlled release of active ingredient preferably have an average release rate of 80% in the time interval between 3 and 20 hours (80% at time measured).

The formulation according to the invention with controlled release of active ingredient preferably have an average release rate of 80% in the time interval between 2 hours and 4 weeks (80% at time measured).

In a particularly preferred embodiment of the medicament formulations with controlled release of active ingredient of the present invention, the formulation has an average release rate of 80% in the period from 3 and 18 hours and an initial release not exceeding 65% of the active ingredient in the first 30 minutes of release.

The admix/formulation/mixture according to the present invention can be formulated so that a relative low initial release of 0 to 30% in the first 30 minutes or a relative high initial release of 30 to 60% of the medicinal substance in the first 30 minutes of medicinal substance release is achieved.

In a preferred embodiment of the admix/formulation/mixture of the present invention is characterized by an average release rate of 80% in the period from 4 to 18 hours, this has a relatively low initial release of 0 to 25% in the first 30 minutes of release.

Another preferred configuration of the medicament formulations with controlled release of active ingredient has an average release rate of 80% in the period from 3 to 16 hours and is distinguished by a relatively high initial release of 35 to 60% in the first 30 minutes of release of active ingredient.

It should be emphasized that the formulation with controlled release of active ingredient of this invention refers to all formulations in which the release of active ingredient is modified so that it takes place with a slower delivery rate than from rapid-release medicinal forms such as, for example, a conventional instillation procedure in the case of bladder cancer treatment Furthermore, the formulation with controlled release of active ingredient of the present invention also include formulations with delayed release in which the delivery of the active ingredient is modified so that the release starts at a later time than with a conventional rapid-release medicinal form. The subsequent release from a delayed-release medicinal form may also take place in controlled fashion with a reduced release rate.

The formulation according to the invention with controlled release of active ingredient also include formulations with pulsatile release, where the delivery of active ingredient takes place intermittently, and formulations in which different principles of controlled delivery of active ingredient are combined.

The formulation of this invention additionally include also medicament formulations which comprise part of the active ingredient in rapid-release form and a further part of the active ingredient in controlled-release form.

The composition provided by the present invention, which furnishes a homogeneous, well-adhered coating of the bladder inner wall, has the capability to provide a prolonged drug release and overcomes the difficulties of adherence to the internal wall of the bladder, such that the chemotherapy drug treatment duration is significantly prolonged and the cancer treatment efficacy is improved.

Beyond maximization of the area of the tissue that is exposed to drug, this coating can also be used to control the distance between organs. Following are several examples:
In the case of application to the bladder wall for the treatment of SBC, the release of drug from the formulation occurs due to two phenomena that take place simultaneously after the gel is applied upon the bladder wall: (1) Drug diffusion from the gel to the aqueous medium (urine); (2) Dissolution of the gel itself in the aqueous medium. We may consider two extreme cases in which the drug either diffuses very fast from the gel to the urine medium, or diffuses very slowly. In the first case, the gel will be depleted very fast of drug so the bladder wall coated by the gel will "see" a low concentration of drug almost from the beginning. On the other hand, the concentration of drug in the urine will be higher and that is the concentration that uncoated parts of the bladder will see. If the case is the second (very slow release rate), the bladder wall coated by the gel will "see" a more or less high concentration of drug for a long time, in fact, until the whole of the gel is dissolved in the urine phase. These two separate but intertwined factors, which we call "two clocks", serve to design different therapies utilizing different drugs and tailor-made polymeric carriers according to the medical needs.

A range of compounds may be utilized to formulate a composition that will render the required properties as described in this invention. Non-limiting example of specific compositions utilized in the present invention are stated below.

In preferred embodiments of the invention, the material comprises polymers and copolymers chosen from among the following: Polycarboxylic acids such as polyglycolic acid polylactic acid and polyacrylic acid; polyurethanes; polyesters such as poly(ethylene terephthalate); polyamides such as nylon; polyacrylonitriles; polyphosphazenes; polylactones such as polycaprolactone; polyanhydrides such as poly[bis(p-carboxyphenoxy)propane anhydride; polyethylene; polyvinyl chloride; ethylene vinyl acetate; Poloxamer block copolymers of the type PEGPPG-PEG; polyethylene oxide (polyethylene glycol); polypropyleneoxide (polypropylene glycol); polymethylacrylate; polysaccharides as dextrin, cyclodextrins, hydroxypropylmethylcellulose (HPMC), hydroxyethylcellulose, hydroxymethylcellulose, chitosan, polyvinyl alcohol and polyvinyl acetate; Polylactide (PLA) and Poly(Lactide-co-Glycolide) (PLGA); polymer microspheres; starch and modified starches; and cellulose polymers.

The described diffusion-controlled or pulsatile formulations can be employed directly and unmodified as medicinal form. However, they may also be further processed, where appropriate with addition of excipients, to the final formulation. In order to achieve a desired release profile it is also possible to combine different coated formulations in one medicinal form, and administration of an initial dose can take place for example by combination with rapid-release formulation particles, e.g. uncoated pellets, granules or powder.

The non-limiting examples presented below illustrate the versatility that is provided by the diverse compositions, that allows its engineering to render different release profiles, flow characteristics, instillation temperatures, coating layer thickness and additional features as required by the specific treatment to be applied upon an internal cavity.

DTCx materials are inserted into the essentially empty bladder using a catheter. The material is inserted at low temperature in the range of 10c to 100c, where the material viscosity is low enough to be able to flow freely into the bladder. As the liquid material flows into the bladder it is naturally heated by the body temperature and eventually it reached that temperature. When the material temperature reaches the gelation temperature - it is passes from the liquid phase to viscous gel phase. The present inevntion is designed to ensure that material will cover essentially most of the surface of the bladder and that it will adhere to the bladder tissue at the point of contact.

According to another embodiment of the present invention, other drugs can be incorporated in the gel composition. Such drugs can be selected from a group consisting of, Antibacterials / Antibiotics, Antiinflammatories / Corticosteroids, Antineoplastics / Cytotoxics, Growth factors such as VEGF (Vascular Endothelial Growth Factor) and Inhibitory factors such as LIF (interleukin 6 class cytokine).

In another preferred embodiment, the hydrogel system is used to introduce one or more cell-cycle modifying agents such as growth factors, interleukin-2, chalones, colcemid, methotrexate or Thymidine for pre-treatment of cancerous tissue. These agents enable the modification of the cell cycle in a way that when chemotherapy agent is subsequently introduced - most of the cancer cells are at the same stage of the cell-cycle and the average cell doubling time is reduced. The result of these modification enhances the cell-kill effect of the chemotherapy and results in a more efficacious treatment.

In another preferred embodiment, the material is formulated with adequate level of pH (approximately 5.5) to dissolve kidney stones, thus enabling the patient to pass the stone naturally. Said material is instilled it into the renal pelvis and left there for several hours to cause dissolution of kidney stones. It is known in the art that most of formed kidney stones can be dissolved in mild acidic solution, but instillation of acidic solution through the bladder, ureter and renal pelvis is not effective, since the solution is excreted immediately.

Using the invented material in this manner will enable improvement of that medical condition with no need for more cumbersome procedures (such as shock-wave lithotripsy).

According to another embodiment of the present invention, at least one of the following is utilized in the admix/formulation/mixture: Poly (propylene oxide) - PPO, Poly (lactide-co-glycolic acid) -PLGA, Poly (N-isopropylacrylamide) - PNIPAM, Poly (propylene fumerate) - PPF, Poly (urethane) - PU, Poly (organophosphazene) - POP, Poloxamers of the type PEO-PPO-PEO (Poly (ethylene oxide), Poly (propylene oxide), Poly (ethylene oxide)) such as poloxamer 68, 88, 98, 108, 124, 127,188, 237, 338 and 407, Stearic Acid, Poly (acrilic acid), Glyceryl Stearate, Cetearyl Alcohol, Sodium Stearoyl Lactylate, Hydroxy-Lenolin or any combination thereof.

According to another embodiment of the present invention the formulation as provided above, can be used as biological glue so as to glue at least two tissues together. By adjusting the viscosity of the formulation such biological glue can be provided.

### EXAMPLE 1

The effectiveness of the present invention hydrogel composition drug delivery to the urinary tract is demonstrated by the following pre-clinical example. Two embodiments of reverse thermal gelation, mucoahdesive and flexible hydrogel mixed with MMC (Kyowa) at a concentration of 1 and 2 mg/ml were prepared as follows.

DTC1: The material comprised Pluronic F-127 Ethylene Oxide/Propylene Oxide Block Copolymer (27.0%); Polyethylene glycol, average MW = 400 (PEG-400) (1.0%); Hydroxypropylmethyl cellulose (HPMC) (0.2%); with the remainder water for injection (71.8%) was mixed with MMC (Kyowa) at a concentration of 1mg/ml.

DTC2: The material comprised Pluronic F-127 Ethylene Oxide/Propylene Oxide Block Copolymer (27.0%); Polyethylene glycol, average MW = 400 (PEG-400) (1.0%); Hydroxypropylmethyl cellulose (HPMC) (0.2%); with the remainder water for injection (71.8%) was mixed with MMC (Kyowa) at a concentration of 2mg/ml.

DTC1 and TDC2 were intravesically instilled in pigs. The MMC concentration in tissue was measured using HPLC and compared to intravesical instillation of 1mg/ml MMC in water (standard treatment of Non-Muscle Invasive Bladder Cancer; Total MMC dosage of 40mg). In addition, bladder condition following instillation and in-vivo gel dissolution rate were evaluated. Higher MMC tissue concentrations were obtained following DTC1 and DTC2 hydrogels treatments in comparison to the standard control treatment (MMC in water). Two hours following intravesical treatment of DTC1 the MMC concentration in urithelium tissue was 11 fold higher for the DTC1 in comparison to that of the control treatment (1mg/ml MMC in water). The MMC concentration following intravesical treatment of DTC2 was 1.8 fold higher than that obtained for TC-3+ 1mg/ml MMC. MMC tissue concentration 6 hr following instillation of DTC1 was 13 fold higher than that of the control treatment. Similar average MMC concentrations were obtained for DTC1 and DTC2. No damages to the urothelium contiguity, or bladder wall perforation were observed. The ureters and urethras were intact. No sign of ureter or urethral obstruction was observed. No clinical effects on animal's vital parameter were detected during the complete treatment duration. In addition, residues of MMC hydrogel were observed in the pig bladder 6 hrs following instillation, supporting release duration of more than 6hr.

### EXAMPLE 2

An in-vitro study was performed to evaluate the efficacy of MMC hydrogel as cytotoxic agents in the urinary bladder. A carcinoma cell line was treated with MMC incorporated into the material disclosed in the present invention, and the results the treatment were compared to treament with MMC dissolved in saline. Viability was assessed by MTT assay which tests mitochondrial function.

Cell lines of human bladder cancer were grown in RPMI 1640 medium, supplemented with FBS, 100 U/ml penicillin and 100mg/ml streptomycin. At Day 1 cells were seeded in 24-well plates at a MMC concentration of 1×10⁴ (four replicates for each concentration, 1 plate for each analysis time). The cells were then exposed for 24 hours to DTC-2; in separate experiments, DTC-2 samples that contained 0.05, 0.1, 0.5 or 1 µg/ml MMC (Kyowa, Hakko Kogyo Co. Ltd.) were used. After 2 hours of incubation, medium was replaced with fresh medium for all plates.

The plates were tested by taking out the medium and adding fresh medium containing MTT reagent. All other plates were returned to the incubator for incubation of 24, 72, 120 and 144h. MTT assays were used to assess toxicity at all time points. The proportion of living cells was calculated by comparison to the control vehicle.

A microplate reader (EL 312e: Bio-Tek Instruments, Winooski VT) was used to evaluate the presence of LDH as a color development measured by absorbance spectroscopy at 450 nm.

Background optical density (OD) was subtracted from the OD readings of a1l samples. Cell viability was calculated by dividing the mean OD absorbance values of the treated wells by the mean OD absorbance of the control wells. All samples were tested in triplicate.

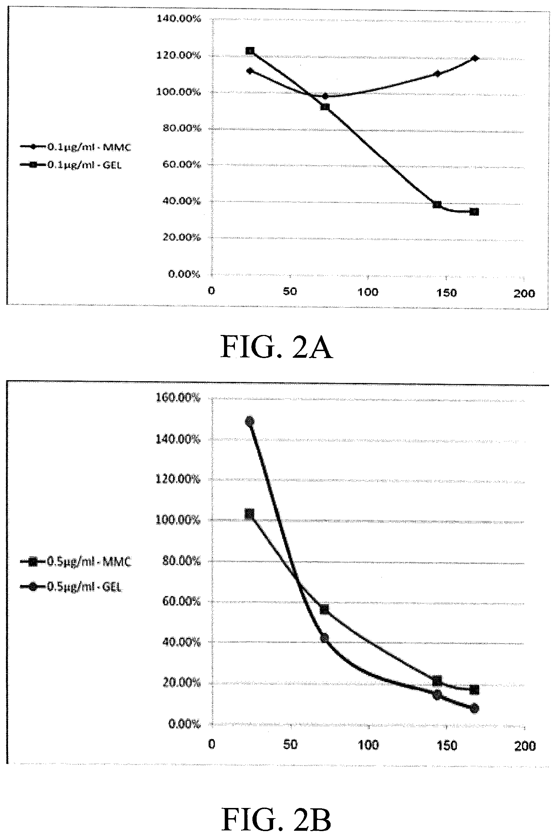

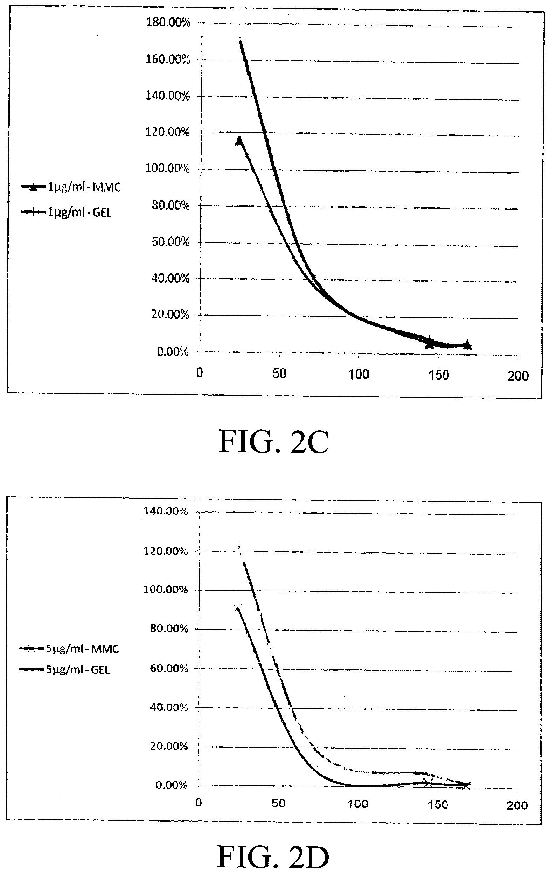

Reference is now made to FIG. 2, which shows graphs of cell viability as a function of time for various experiments. These results clearly demonstrate MMC cytotoxicity when different MMC concentrations are used in saline and in TC-2 respectively.

These studies clearly demonstrate that a dramatic effect may be observed in the case of 0.1 µg/ml (see FIG. 2A) where the difference between the effect of the DTC-2 hydrogel and that of the MMC-saline solution is most pronounced. For MMC-saline solution, there is practically no cell-kill, while with DTC-2 the cell-kill effect reaches 60%.

A direct relationship was demonstrated between MMC time of exposure and cytotoxicity. As seen in the graphs, in practically all cases the general trend is that the cytotoxicity increases as exposure time increases.

A direct relationship was also demonstrated between MMC concentration and cytotoxicity. Comparing the graphs obtained for different concentrations shows a higher cytotoxicity effect with the increase of concentration.

Thus, higher concentrations of MMC and longer exposure contribute significantly to a higher cytotoxicity of MMC. When exposure time was increased from 2 hours to 24 hours, a cell kill level was achieved with less than one third of the MMC dose used for two hours exposure.

MMC concentrations in intravesical instillations have been evaluated in many different studies. Current treatment concentration is the highest that can be tolerated by the patient. Our results demonstrate that a further improvement in MMC efficacy can be gained by increasing the exposure time to MMC even at lower MMC concentrations.

### EXAMPLE 3

En additional example for the effective drug delivery to the urinary tract of present invention composition was demonstrated by a preclinical study testing the delivery of Gemzar chemotherapy. The study was conducted using sick female mice model and tested Gemzar hydrogel effect on a MBT-2. Gemzar hydrogel composition was included (1mg Gemzar mg/ml gel composed of 27.0% PF 127; 1.1% PEG-400, 0.2% HPMC. MBT-2 tumors are extremely aggressive and resistant to chemotherapy therefore somewhat differ from human superficial bladder cancer. In this study, treatment was applied 14, 10, and 3 days following cell implantation. Each group was divided into a Gemzar hydrogel arm and a standard instillation arm consisting of Gemzar in saline, which served as control group. The results showed that the procedure was safe (indeed, with low mortality) and efficient, that is, consistent lower weights of cancerous bladder in the Gemzar hydrogel arm were obtained as compared to the control arm.

### EXAMPLE 4

An additional example for the effective drug delivery to the urinary tract of the present invention composition was demonstrated by a preclinical study testing the delivery of botulinium toxin A (BoNT-A) to rats bladder induced with chronic cystitis.

The effect of BoNT-A mixed with one embodiment of the invented material DTC-4 on bladder cystitis was demonstrated in a rat model (Sprague-Dawley) by comparing the effect of intravesical administration of BoNT-A in saline and in gel. The BoNT-A gel formulation (DTC-4) tested included 20U BoNT-A /ml gel composed of 27% Pluronic F127, 0.2% HPMC and 1% PEG-400 in water. Intravesical instillation of just DTC-4 and of saline with no botullinium toxin served as control groups. Chronic cystitis was induced by intraperitoneal injection of Cyclophosphamide (75 mg/kg was injected on day 1, 4 and 7). On day 2 PE-50 tubing was inserted into the rat bladder through the urethra. The bladder was emptied of urine and filled with DTC-4 or saline with and without BoNT-A (1ml, 20u/ml Allergan). On day 8 the animals were anesthetized and their bladders were examined by cystometrogram recording via PE-50 tube inserted into the bladder dome. After recovery the animals were gently restrained and the suprapubic catheter was connected to infusion pump (0.08 ml/min continuously) and pressure transducer for recording intravesical pressure and for saline infusion into the bladder to elicit repetitive voiding. The amplitude and the inter-contraction interval of reflex bladder contractions were recorded. Each tested groups included 2-4 rats.

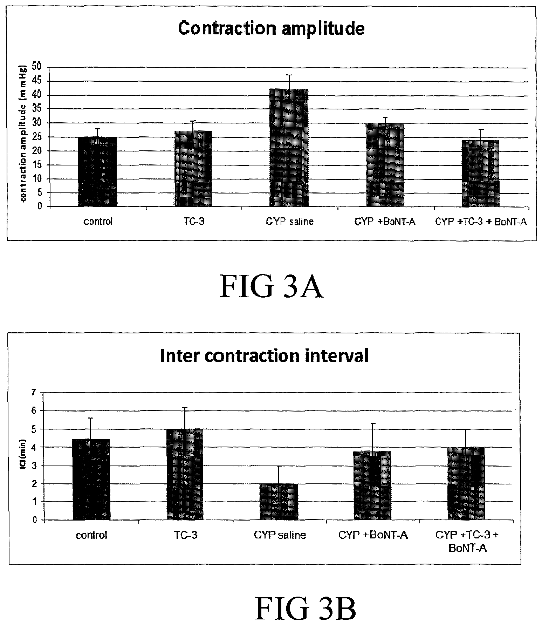

The bladder contraction amplitude measured is shown in FIG. 3. The results demonstrate that following CYP administration the bladder contraction amplitude increased from 25-27 mmHg (contraction level for rat bladder with no hyperactivity) to about 42 mmHg. Following administration of BoNT-A in saline and in TC-3 gel to rats with induced chronic cystitis the contraction decreased to the baseline level amplitude of 30 mmHg and 24 mmHg, respectively. Better response was received for the BoNT-A in gel treatment demonstrating the activity of the BoNT-A in gel and efficacy of BoNT-A mixes with gel treatment.

Similar results were obtained for the measurements of bladder inter- contraction intervals summarized in Figure 2. Following CYP administration the bladder inter- contraction intervals were shown to decrease from 4-5 min for rat administered with saline or gel to 2 min. Intravesical administration of BoNT-A in saline and in TC-3 gel to rats with induced chronic cystitis increased the inter-contraction intervals to about 4 min, close to the inter-contraction intervals for bladder with no hyperactivity. These results further support the activity of the BoNT-A in the gel and the efficacy of the BoNT-A mixes with gel treatment.

### EXAMPLE 5

The present invention composition for effective drug delivery to the urinary tract includes reverse thermal gelation, mucoahdesive and flexible hydrogel with slow releasing capabilities. This special combination of properties is essential for ensuring effective drug delivery to the urinary tract. Examples for each one of the required properties are enclosed:

### Mucoadhesiveness

The mucoadhesivness properties of one embodiment (DTC1) of the reverse thermal gelation hydrogel to the mucosal tissue was demonstrated using the "rolling ball" method for tackiness testing according to the ASTM D-3121-94 and to peel strength according for adhesiveness testing according to ASTM standard D-2256-03. In these experiments, the tackiness and adhesiveness to pig bladder tissue of DTC1 was compared to the adhesiveness of compositions disclosed in U.S. Pat. No. 6,207,180; U.S. Pat. No. 6,894,071; and U.S. Pat. Appl. US2006/0127210. The results summarized in Table 1 demonstrate substantially higher the tackiness and adhesiveness of DTC1 (about 20 times higher) in comparison to the materials known in the prior art.

**Table 1: comparison of tackiness using "rolling ball" and "Peel strength" method**

| Composition | "rolling ball" tackiness ASTM D-3121-94 and PTSC-6 standard | | "Peel strength" Adhesiveness according to ASTM standard D-2256-03 |
|---|---|---|---|
| | Test 1(cm) | Test 2(cm) | Adhesiveness (N/cm²) |
| U.S. Pat. No. 6,207,180 | 32 | 20 | 0.068 |
| U.S. Pat. No. 6,894, 017 | >45 | >28 | 0.047 |
| U.S. Pat. Appl. 2006/0127420 | >45 | >28 | 0.090 |
| DTC1 | 1.0 | 1.6 | 1.77 |

A second set of in vitro tests of the bioadhesive properties of the material disclosed herein was performed. These tests were performed using wet swine bladder tissue simulating in-vivo tissue conditions. Measurements were performed using TAXT2 texture analyzer according to the following protocol.

A tissue specimen was placed on a foam tape mounted onto the cylindrical support of the instrument (2 cm diameter and 4 cm length) and secured with a string. The whole support was then positioned at the top of the measuring system and held in place by a clamp. A given weight of hydrogel (for example, 0.5 g) was evenly poured onto another support of similar dimensions. The support was then affixed on the lower probe of the instrument. The two supports were aligned to ensure that the gel comes into direct contact with the surface of the swine tissue when the upper support is lowered. Measurements were performed at 25°C.

Before measurement, 100 µl of simulated urine fluid was evenly spread on the surface of the tissue. The upper support was then lowered at a speed of 0.5 mm/s to contact with the gel at a force of 1 N for a contact time of 10 s. It was then withdrawn at a rate of 1.0 mm/s to a distance of 10 mm. An acquisition rate of 200 points/s was chosen for the analysis. Data collection and calculation can be performed using the XTRA Dimension software package of the instrument. The work of adhesion and peak detachment force were used to evaluate the bioadhesive strength of the films. The work of adhesion is calculated from the area under the force-distance curve, and the peak detachment force is taken as the maximum force needed for detaching the film from the tissue. All measurements were performed in triplicate. The results are summarized in Table 2.

**Table 2. In vitro bioadhesive strength measured using wet bladder tissue**

| Formulation | Pluronic F127 | HPMC | PEG 400 | Double Distilled Water | Work of Adhesion (mJ) | Peak Detachment Force (N) |
|---|---|---|---|---|---|---|
| A | 25.0% | 0.1% | 0.5% | balance | 0.51 | 3.34 |
| B | 27.0% | 0.1% | 1.0% | " | 0.73 | 4.43 |
| C | 27.0% | 0.2% | 1.0% | " | 1.13 | 6.35 |

A marked increase in the bioadhesion strength was observed with an increase in HPMC content.

### EXAMPLE 6

### Flexibility

In order for the hydrogel formulation to be suitable for drug delivery in the bladder it must comply with the natural physiological changes in volume and shape i.e. expansion during urine accumulation and contraction during urine voiding. During its the physiological function the bladder expands to about five times its original size.

In order to estimate the minimum flexibility of the various embodiments of the hydrogels used in the present invention, and the effect of tissue tension and expansion on hydrogel coverage, an in vitro test method was developed. A hydrogel according to the present invention stained with methylene blue was layered on top of the mucosal side of a square section of bladder tissue 3 cm² by 3 cm². Following hydrogel gelation, the tissue was stretched and expanded by 3 fold to each direction (horizontally and vertically). The received area was thus 9 fold larger than the initial surface tissue area. The hydrogel coverage of the tissue following tissue expansion was examined. The hydrogel was shown to follow the stretched tissue. No gel detachment was observed for any of the hydrogel formulations disclosed herein.

### EXAMPLE 7

### Reverse thermal gelation:

Gel point measurements were made for a number of embodiments of reverse thermal hydrogels disclosed in the present invention composition for effective drug delivery. The general procedure for the measurements was as follows. First, 50 ml of the material was poured into a 100-ml glass container. A TEFLON-coated magnet of about 2-2.5 cm length was then placed in the container. A thermocouple was then inserted into the bulk of the hydrogel. The container was placed in an ice bath on a magnetic stirrer plate. After the temperature of hydrogel dropped to 5°C, the ice bath was removed from the magnetic stirrer and the glass container containing the hydrogel was placed directly on top of the magnetic stirrer plate. The magnetic stirrer was then turned on and run at medium speed (about 120 rpm). The temperature was allowed to rise gradually (∼1°C/min) to room temperature. The gel point for a particular measurement was recorded as the temperature at which the magnet stopped rotating. For each sample, the entire procedure was performed twice, and the gel point determined as the average of the two measurements.

The gel point was measured for three different embodiments of the material disclosed herein. The results of the measurements are summarized in Table 3.

**Table 3**

| Formulation | Pluronic F127 | HPMC | PEG 400 | Double Distilled Water | Gel Point (°C) |
|---|---|---|---|---|---|
| A | 25.0% | 0.1% | 0.5% | balance | 16.5 |
| B | 27.0% | 0.1% | 1.0% | " | 14.1 |
| C | 27.0% | 0.2% | 1.0% | " | 11.9 |

A further set of tests was performed in which MMC was incorporated into the material at a typical dosage concentration in order to determine the effect of the MMC (and its accompanying excipient) on the gel point of the final composition. Formulation D includes pure MMC; formulation E includes commercially obtainable MMC that includes 2 parts mannitol per 1 part MMC (for example, Boehringer Mannheim) and Formulation F includes 24 parts of sodium chloride per 1 part MMC (for example, Kyowa). The results are summarized in Table 4.

**Table 4**

| Formulation | Pluronic F127 | Pluronic F68 | HPMC | PEG 400 | MMC | NaCl | Mannitol | Gel Point (°C) |
|---|---|---|---|---|---|---|---|---|
| D | 27.0% | - | 0.2% | 1.0% | 0.1% | - | - | 11.8 |
| E | 27.0% | - | 0.2% | 1.0% | 0.1% | - | 0.2% | 11.5 |
| F | 27.0% | - | 0.2% | 1.0% | 0.1% | 2.4% | - | 5.1 |
| G | 20.0% | 10% | 0.2% | 1.0% | 0.1% | 2.4% | - | 24.0 |

As can be seen, incorporation of NaCl into the commercial MMC significantly lowers the gel point while mannitol does not affect the gel point very much. The drop in gel point due to addition of NaCl can be compensated by incorporation of Pluronic F68 in the hydrogel formulation as demonstrated in formulation G.

In addition to the gelation point of the reverse thermal gelation hydrogel its viscosity as a function of temperature is an important feature that determines the hydrogel's behavior and ability to perform properly. Therefore the rheological properties of formulation candidates were studied. The viscosity vs temperature curves and values of Tg (gelling temperature) for each formulation were determined by RV DV III Brookfield rheometer and DVII LV viscometer. The temperature at which a sharp increase in viscosity was obtained was determined as Tg. The viscosity measurement of the solid state of the gel (after complete solidification) was out of the range of the used rheometer and viscometer.

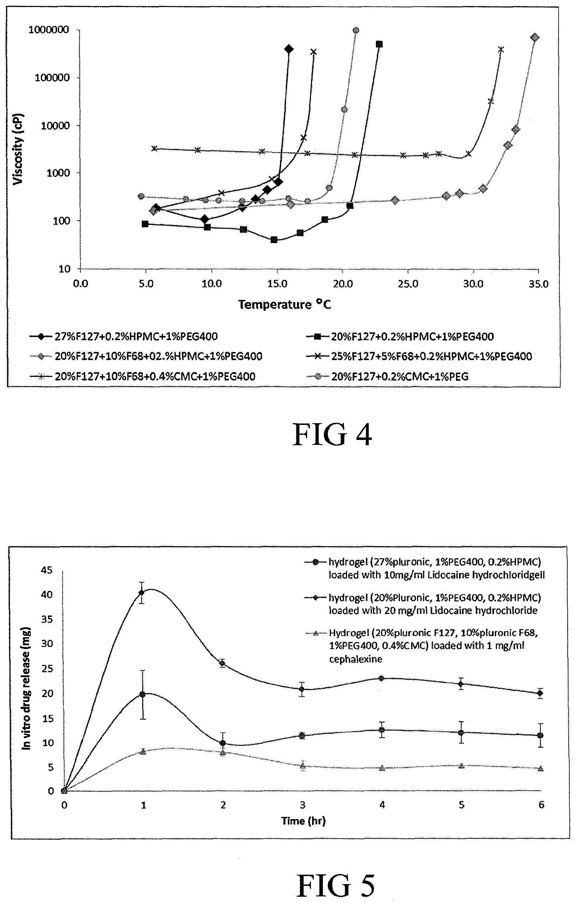

Reference is now made to FIG. 4, which presents a semi-logarithmic graph of viscosity as a function of temperature over a range of 5-35 °C for six different embodiments of reverse thermal hydrogels disclosed in the present invention composition for effective drug delivery (TCA, TCB, TCC, TCD, TCE, TCF), and Table 5, which presents the results of a sample gel point measurements.

**Table 5**

| Formulation | Pluronic F127 | Pluronic F68 | HPMC | CMC | PEG-400 | Gel Point |
|---|---|---|---|---|---|---|
| TCA | 27.0% | - | 0.2% | - | 1.0% | 15°C |
| TCB | 20.0% | - | 0.2% | - | 1.0% | 22°C |
| TCC | 20.0% | 10.0% | 0.2% | - | 1.0% | 33°C |
| TCD | 25.0% | 5.0% | 0.2 | | 1.0% | 17°C |
| TCE | 20.0% | 10.0% | - | 0.4% | 1.0% | |
| TCF | 20.0% | - | - | 0.2% | 1.0% | 20°C |

This information is fundamentally important for the design and engineering of the hydrogel device since viscosity characteristics are critical in the gel flow through the catheter and spreading upon the bladder inner surface as it is injected by means of the catheter.

### EXAMPLE 8

### Slow releasing capabilities

Studies performed by the inventors demonstrate that controlled release of the drug can be obtained which considerably prolong the actual contact between the bladder wall and the drug. This slow-release effect, which can take up to 24 hours, dramatically increases such contact time and thus the continuous therapeutic effect of the drug upon the cancerous tissue (in case chemotherapeutic drug is delivered). This effect, in tandem with the creation of an effective, stable coating upon the bladder wall, is expected to render a superior therapeutic effect in the treatment of urinary tract diseases including bladder cancer.

The inventor has performed numerous in vitro release experiments demonstrating the slow release of loaded drugs from several gels embodiments. Among drugs that were tested were chemotherapeutic drugs (Mitomycin C, Gemzar), local anesthetic drug (Lidocaine hydrochloride) and antibiotic drug (Cephalexin). All results demonstrated gradual release of the drug embedded in the hydrogels composition in conditions similar to those of a human bladder (37 °C, urine medium). The in-vitro release experiments were performed using membraneless dissolution model: The hydrogel was loaded with the tested drug, layered in a glass containers, and equilibrated in the experimental temperature until homogenous gel layer was formed. Artificial urine used as acceptor solution was equilibrated at the experimental temperature was added. The glass containers (with gel and medium) were placed in a shaking water bath at a constant temperature of 37°C. Acceptor solution was discarded every hour and replaced by a fresh solution. Drug concentration in acceptor solution was measured spectrophotometrically, gel dissolution was determined gravimetrically. Two examples are enclosed:
a. Reverse thermal gelation hydrogel composed of Pluronic F-127 Ethylene Oxide/Propylene Oxide Block Copolymer (27.0%); Polyethylene glycol, average MW = 400 (PEG-400) (1.0%); Hydroxypropylmethyl cellulose (HPMC) (0.2%); and the remainder water for injection (71.8%) was loaded with at a concentration of 10 mg/ml and 20 mg/ml Lidocaine hydrochloride
   The in vitro release of Lidocaine hydrochloride (10 and 20 mg/ml) from the hydrogel formulation enclosed above is shown in FIG 5. The released amount of Lidocaine hydrochloride was twofold higher for hydrogel loaded with 20 mg/ml Lidocaine hydrochloride in comparison to the hydrogel loaded with 10 mg/ml Lidocaine hydrochloride formulation. In addition, higher amount of Lidocaine hydrochloride was released from both formulations to the acceptor solution in the first testing time point (1hr). The amount of Lidocaine hydrochloride released in the following time points was relatively constant.
b. Reverse thermal gelation hydrogel composed of Pluronic F-127 (20.0%); Pluronic F-68 (10.0%) Polyethylene glycol, average MW = 400 (PEG-400) (1.0%); carboxymethyl cellulose soidium (CMC) (0.4%); and the remainder water for injection (68.8%) was loaded with at a concentration of 1 mg/ml cephalexin

The in vitro release of cephalexin (1 mg/ml) from the hydrogel formulation enclosed above is shown in FIG 5. The results demonstrate constant release of cephalexin at 3-6 hrs.

### EXAMPLE 9

### Delivery duration control

An important feature that influences the hydrogel is its dissolution rate/ erosion rate. Therefore dissolution properties of hydrogels disclosed in the present invention composition were studied.

The dissolution rate was determined using an *in-vitro* membrane free dissolution model at 37°C. In this model the gel is dissolved into the receptor medium (Dulbecco's phosphate buffer saline) which is carefully layered on top of a smooth and flat gel layer. The amount of gel dissolved is determined gravimetrically. After each sampling of the receptor medium it is carefully discarded and replaced by a fresh medium.

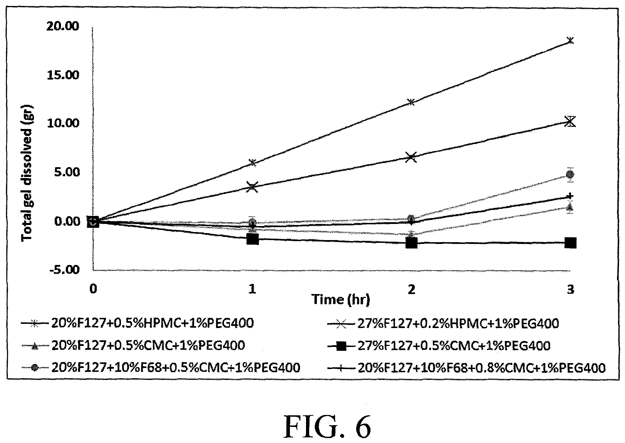

Reference is now made to FIG. **6****,** which presents the dissolution rate of six different hydrogels herein disclosed. The results demonstrate different dissolution rate of the various formulations tested. These various dissolution rates will determine the treatment duration following instillation of the drug loaded hydrogel. Hence, the reverse thermal gelation hydrogel composition for effective treatment of the urinary tract will be set based on the beneficial treatment duration for specific disease.

### EXAMPLE 10

The following are further examples of mixtures according to the present invention:
10.1 - Vitamin gel for topical action provided, according to the following:

| | |
|---|---|
| Vitamin (A, D or K) 0.05 %w/w Lecithin | 12 % |
| PEG 800 | 1 % |
| Isopropyl stearate | 8 % |
| Pluronic F-127 | 20 % |
| Double distilled water (DDW) | to 100 % |

10.2 - Paclitaxel gel for cancer treatment, according to the following:

| | |
|---|---|
| Paclitaxel | 0.1% |
| Pluronic F-127 | 10.0% |
| Isopropyl Palmitate | 1.0% |
| Lecithin | 0.8% |
| Sodium Acryloyldimethyl | |
| -Taurate Copolymer | 1.2% |
| Sorbic Acid | 1.0% |
| Potassium Sorbate | 0.1% |
| DDW | to 100% |

10.3 - Oral anesthetic gel, according to the following:

| | |
|---|---|
| Lidocaine | 1.0 % |
| Pluronic F-127 | 27 % |
| Ethoxyl diglycol | 10 % |
| Lecithin | 2.0 % |
| Mint flavor | 0.1 % |
| DDW | to 100% |

10.4 - Anti-inflammatory composition, according to the following:

| | |
|---|---|
| PEG-PLGA-PEG* | 24.5 % |
| HPMC | 0.2 % |
| PEG 400 | 0.5 % |
| Ibuprofen | 0.2 % |
| DDW | to 100% |

| | |
|---|---|
| *Ethylene glycol-lactic acid-co-glycolic acid-ethylene glycol triblock copolymer | |

10.5 - Antibacterial gel, according to the following:

| | |
|---|---|
| PEG 400 | 1.5% |
| PEG 1200 | 12.0% |
| Polysorbate 60 | 6 % |
| Pluronic F-127 | 22 % |
| Polyvinyl pirrolidone - iodine complex | 5.5% |
| DDW | to 100% |

10.6 - Antibiotic gel, according to the following

| | |
|---|---|
| Pluronic F-127 | 20 % |
| Pluronic F-68 | 10 % |
| Carboxymethyl cellulose sodium | 0.5% |
| PEG 400 | 1% |
| Cephalexin | 0.1% |
| 50mM Tris-HCL buffer pH=8 | |
| WFI | to 100% |

10.7 - MMC gel, according to the following

| | |
|---|---|
| Pluronic F-127 | 20 % |
| Carboxymethyl cellulose sodium | 0.5% |
| PEG 400 | 1% |
| MMC | 0.2% |
| In 50mM Tris-HCL buffer pH=8 | to 100% |

10.8 - Botox gel, according to the following

| | |
|---|---|
| Pluronic F-127 | 20 % |
| Carboxymethyl cellulose sodium | 0.4% |
| PEG 400 | 1% |
| Botox | 5U/gr |
| In 50mM buffer acetate pH=5 | to 100% |

### EXAMPLE 11

A further embodiment of the material disclosed in the present invention (DTC-11) is utilized upon organ linings consisting of serous tissue that does not possess a mucosal layer, in particular pleural and peritoneal walls. A condition common in several lung and heart diseases is pleural effusion, when serous fluids, pus or chyle accumulate into the space between the visceral pleura and the parietal pleura layers surrounding the lungs - condition called also hydrothorax. Certain malignancies enlarge the space between pleura layers and cause excessive levels of fluids to accumulate and impair breathing. Standard treatments for pleural effusions is the insertion of intercostals drain, often accompanied by surgical pleurodesis - in which the two pleural surfaces are scarred to each other so that no fluid can accumulate between them. Surgical joining of the layers is not always successful, but it is permanent.

A material of the present invention can be administered into the pleura cavity to adhere to the pleura layers and provide both a mechanical bond and sustained release of drug for treatment of the underlying malignancy (e.g. Tetracycline antibiotic for bacterial infection, or NSAID such as Naproxen to treat fever and inflammation).

The pleura-hydrogel material is inserted into the pleura space via a catheter or trocar, as non-viscous liquid at a temperature that is below 20 °C and adheres to the surrounding tissue as it heats to body temperature. The material is designed to dissolve gradually into the pleura fluids over less than a week and both maintain mechanical support and release therapeutic agents during that whole period.

This procedure can replace the more invasive pleurodesis with the additional benefits of tissue damage reduction, the soothing effect of the hydrogel and improved healing due to the sustained release of anti-inflammatory agents.

The following exemplary formulation can be applied (DTC11):

| | |
|---|---|
| PEG-PLGA-PEG* | 24.5 % |
| HPMC | 0.1 % |
| PEG 400 | 0.4 % |
| DDW | to 100% |

| | |
|---|---|
| *Ethylene glycol-lactic acid-co-glycolic acid-ethylene glycol triblock copolymer | |

Said composition adheres well to said walls providing an effective matrix for the transport and release of therapeutic active ingredients. In particular, the admixture can carry anti-inflammatory drugs to be applied in case of pleural or peritoneal inflammation.

### EXAMPLE 12

Fixation of organs and prevention of tissue adhesion in the abdomen during laparoscopy:
A material of the current invention can be introduced into the abdomen cavity and provide mechanical support to the target organs in the position that best fits the surgical procedure. The peritoneum-hydrogel material can be inserted into the peritoneum cavity via endoscope working channel, a catheter or trocar, as non-viscous liquid at a temperature that is below 15 °C and adhere to the surrounding tissue as it heats to body temperature. The material is designed to dissolve gradually into the pleura fluids over less than a week and both maintain mechanical support of the organs for several hours and release the drugs during a longer period.

Similar method, but with different materials can be used to prevent the adhesion of tissues between organs in the treated area, which may often occur during laparoscopic surgery.

The main advantages of this method are the combination of its ability to replace a more invasive procedure, the reduction of tissue damage, the soothing effect of the hydrogel and the enhanced healing effect of the sustained release of anti-inflammatory agents. A further advantage is the prevention of the need to remove the fixation surgically because of the natural degradation and expelling of the invented material from the treated area.

The following exemplary formulation can be applied (DTC-12):

| | |
|---|---|
| PEG-PLGA-PEG* | 24.5 % |
| CMC | 0.4 % |
| PEG 400 | 0.4 % |
| DDW | to 100% |

### EXAMPLE 13

A binary API system (DTC-13 to DTC-16) was formulated which allowed a different release profile from the same gel structure. The relative release rate from the basic formulation was controlled by the addition of changing amounts of the surfactant sodium dodecyl sulfate (SDS), which affected the overall lipophilic/hydrophilic balance of the formulation. The APIs in question were mitomycin C (MMC) and lidocaine. The following table presents the composition of three basic formulations and the release time of 80% of each of the two APIs. From an applicative point of view, it is of much interest to obtain a relatively rapid release of lidocaine, which produces the local anesthetic effect upon the organ tissue to be treated, followed by a slow release of the MMC, that may allow a superior chemotherapeutic treatment of said organ.

| MATERIAL | DTC-13 | DTC-14 | DTC-15 | DTC-16 |
|---|---|---|---|---|
| Pluronic F127 (%) | 27.0 | 27.0 | 27.0 | 27.0 |
| HPMC (%) | 0.2 | 0.2 | 0.2 | 0.2 |
| PEG-400 (%) | 1.0 | 1.0 | 1.0 | 1.0 |
| SDS (%) | 0 | 0.05 | 0.1 | 0.2 |
| DDW (%) | 71.8 | 71.75 | 71.7 | 71.6 |
| MMC (%) | 0.1 | 0.1 | 0.1 | 0.1 |
| Lidocaine (%) | 1.0 | 1.0 | 1.0 | 1.0 |
| Release time 80% MMC (hrs) | 16 | 15 | 14 | 12 |
| Release time 80% Lidocaine (min) | 180 | 120 | 60 | 10 |

The table above shows that adding a small amount of SDS dramatically affects the release profile of the two drugs respectively. This exemplifies, without limiting, the possibilities of engineering the gel composition in a way that allows two or more different APIs to release from the gel matrix each at their own pace - according to the treatment needs. In the example above, it may be desirable, for example, that the soothing effect of the lidocaine be felt by the patient rapidly, just before the MMC commences its own activity, that may be painful, and thus a rapid release of the lidocaine may be desired while a slow release of the same may be unfelt and this ineffective. On the other hand, one can conceive other treatments where a lower but more prolonged anesthetic effect will be required, and thus the formulation will change accordingly.

## Claims

1. A thermoreversible hydrogel composition comprising at least one therapeutic agent selected from the group consisting of antineoplastic agents, chemotherapeutic agents, anti-infective agents, antimicrobial agents, antiparasitic agents, antiviral agents, agents acting on the blood, antihemorrhagics, antithrombotic agents, antifungals antiseptics, agents for treating diseases of the genito-urinary system, anti-inflammatory agents, neurological agents, gene therapy agents, corticosteroids, analgesic and anesthetic agents, growth factors, VEGF, inhibitory factors, LIF, proteins, mucin, cell cycle modifying agents for synchronization of cell cycle selected from the group comprising chalones, colcemid, methotrexate, and thymidine, and any combination thereof, for use in the treatment of the urinary tract, wherein the therapeutic agent is delivered to the urinary tract; **characterized in that** said thermoreversible hydrogel composition comprises a thermoreversible hydrogel **characterized by**:
a viscosity of less than 5 Pa·s over a temperature range of 4 °C - 12 °C;
a viscosity of greater than 10³ Pa·s at 37 °C;
a "peel strength" adhesiveness according to ASTM standard D2256-03 of 0.5 - 5.0 N cm⁻² at 37 °C; and,
flexibility sufficient such that a 3 cm² × 3 cm² section of bladder tissue layered with said thermoreversible hydrogel at room temperature can be stretched to 9 cm² × 9 cm² without detachment of said thermoreversible hydrogel from said bladder tissue, and
wherein said thermoreversible hydrogel comprises a composition comprising a poloxamer.

2. A thermoreversible hydrogel composition comprising at least one therapeutic agent selected from the group consisting of antineoplastic agents, chemotherapeutic agents, anti-infective agents, antimicrobial agents, antiparasitic agents, antiviral agents, agents acting on the blood, antihemorrhagics, antithrombotic agents, antifungals antiseptics, agents for treating diseases of the genito-urinary system, anti-inflammatory agents, neurological agents, gene therapy agents, corticosteroids, analgesic and anesthetic agents, growth factors, VEGF, inhibitory factors, LIF, proteins, mucin, cell cycle modifying agents for synchronization of cell cycle selected from the group comprising chalones, colcemid, methotrexate, and thymidine, and any combination thereof for use in the treatment of a mucosal or serous membrane **characterized in that** said thermoreversible hydrogel composition comprises a thermoreversible hydrogel **characterized by**:
a viscosity of less than 5 Pa·s over a temperature range of 4 °C - 12 °C;
a viscosity of greater than 10³ Pa·s at 37 °C;
a "peel strength" adhesiveness according to ASTM standard D2256-03 of 0.5 - 5.0 N cm⁻² at 37 °C; and,
flexibility sufficient such that a 3 cm² × 3 cm² section of bladder tissue layered with said thermoreversible hydrogel at room temperature can be stretched to 9 cm² × 9 cm² without detachment of said thermoreversible hydrogel from said bladder tissue, and
wherein said thermoreversible hydrogel comprises a composition comprising a poloxamer.

3. The composition for use according to claim 1 or 2, wherein said thermoreversible hydrogel additionally comprises at least one tight junction modifier / cell membrane permeability enhancer.

4. The composition for use according to claim 3, wherein at least one of the following is true:
said tight junction modifier / cell membrane permeability enhancer is selected from the group comprising anionic surfactants, non-anionic surfactants, charged polymers, dimethyl sulfoxide (DMSO), decylmethyl sulfoxide, tert-butyl cyclohexanol, fatty acids their esters and salts, ethanol, nicotinamide, urea, perfluoropolyether, monoterpene ketones, disodium citrate, succinic acid and tris;
said tight junction modifier / cell membrane permeability enhancer comprises a surfactant selected from the group comprising polysorbates, sodium dodecyl sulfate, and dextran sulfate;
said tight junction modifier / cell membrane permeability enhancer comprises a charged polymer selected from the group comprising chitosan, poly-arginine, polylysine, and aliginate; and,
said tight junction modifier / cell membrane permeability enhancer comprises a monoterpene ketone selected from the group comprising (-) menthol, (-) menthone, peppermint oil, and spearmint oil.

5. The composition for use according to any one of claims 1 - 4, wherein said thermoreversible hydrogel additionally comprises at least one component selected from the group comprising adhesive and thickening compounds; bonding agents; pH-modifying substances; diffusion coatings; plasticizers; components for increasing permeability within the formulation; swellable excipients; matrix forming polymers; and any combination thereof.

6. The composition for use according to claim 5, wherein at least one of the following is true:
said adhesive and thickening compounds are selected from the group comprising gums;
said adhesive and thickening compounds are selected from the group comprising guar gum, locust beam gum, xanthan gum and their derivatives;
said bonding agents are selected from the group comprising polycarbophil, cellulose, microcrystalline cellulose, cellulose derivatives, HPMC, hydroxypropylcellulose (HPC), low-substituted hydroxypropylcellulose, dicalcium phosphate, lactose, sucrose, ethylcellulose, hydroxypropymethylcellulose acetate succinate (HPMCAS), polyvinylpyrrolidone (PVP), vinylpyrrolidone/vinyl acetate copolymer, polyethylene glycol, polyethylene oxide, polymethacrylates, polyvinyl alcohols (PVA), partially hydrolysed polyvinyl acetate (PVAc), polysaccharides, hyaluronic acid, fats, fatty acid derivatives, and any combination thereof;
said pH-modifying substances are selected from the group comprising acids, bases, and buffers;
said pH-modifying substances are selected from the group comprising adipic acid, malic acid, L-arginine, ascorbic acid, aspartic acid, benzenesulphonic acid, benzoic acid, succinic acid, citric acid, ethanesulphonic acid, 2-hydroxyethanesulphonic acid, fumaric acid, gluconic acid, glucuronic acid, glutamic acid, potassium hydrogen tartrate, maleic acid, malonic acid, methanesulphonic acid, toluenesulphonic acid, trometamol, tartaric acid, potassium hydrogen tartrate and their respective salts, phosphate salts, and Tris;
said diffusion coatings are selected from the group comprising ethylcelluloses and polymethacrylates, cellulose acetate and cellulose acetate butyrate or any combination thereof;
said components for increasing permeability within the formulation are selected from the group comprising polyethylene glycols, PVP, PVA, HPMC, HPC, hydroxyethylcelluloses (HEC), methylcellulose (MC), carboxymethylcelluloses and their salts, dextrins, maltodextrins, cylcodextrins, dextrans, urea, salts, sugars, sugar alcohols, and any combination thereof;
said swellable excipients are selected from the group comprising polyvinylpyrrolidones, crospovidones, crosslinked sodium carboxymethylcellulose, crosslinked sodium carboxymethylstarch, polyethylene oxides, polymethyacrylates, L-HPC, cellulose acetate, ethylcellulose, polymethacrylates, high-molecular weight polyethylene oxides, xanthan gum, copolymers of vinylpyrrolidone and vinyl acetate, polyvinylpyrrolidones, crospovidones, crosslinked sodium carboxymethylcellulose, crosslinked sodium carboxymethylstarch, poly(hydroxyalkyl methacrylate), alginates, galactomannans, and any combination thereof; and,
said matrix forming polymers are selected from the group comprising hydroxyethylmethylcelluloses, HPC, HEC, MC, ethylcelluloses, alkylcelluloses, hydroxyalkylcelluloses, hydroxyalkylmethylcelluloses, sodium CMCs, alginates, galactomannans, xanthans, polyethylene oxides, polyacrylic acids, polymethacrylic acids, polymethacrylic acid derivatives, polyvinyl alcohols, partially hydrolysed polyvinyl acetate, polyvinylpyrrolidone, agar, pectin, gum arabic, tragacanth, gelatin, starch, starch derivatives poly(propylene oxide) (PPO), poly(lactide-co-glycolic acid) (PLGA), poly(N-isopropylacrylamide) (PNIPAM), poly(propylene fumarate) (PPF), polyurethane (PU), poly(organophosphazene) (POP), stearic acid, poly(acrylic acid), glyceryl stearate, cetearyl alcohol, sodium stearoyl lactylate, hydroxy-lanolin, and any combination thereof.

7. The composition for use according to any one of claims 1 - 6, wherein said thermoreversible hydrogel is additionally used as a biological glue.

8. The composition for use according to any one of claims 1 - 7, wherein said thermoreversible hydrogel is further **characterized by** at least one characteristics selected from the group comprising:
a viscosity of less than 0.5 Pa·s over a temperature range of 4 °C - 12 °C;
a viscosity of greater than 3 × 10³ Pa·s at 37 °C;
a "peel strength" adhesiveness according to ASTM standard D2256-03 of 1.0 - 5.0 N cm⁻² at 37 °C; and,
flexibility sufficient to permit the volume of the tissue to which said composition is applied to expand by a factor of at least 3 without detachment of said gel from said tissue.

9. The composition for use according to any one of claims 1 - 7, wherein said thermoreversible hydrogel comprises a composition selected from the group comprising of:
(i) between 20% and 30% (w/w) ethylene oxide/propylene oxide block copolymer, between 0.05% and 0.5% (w/w) hydroxypropylmethylcellulose (HPMC), between 0.1% and 2.5% (w/w) polyethylene glycol (PEG)-400, and the balance water;
(ii) between 20% and 30% (w/w) ethylene oxide/propylene oxide block copolymer, between 0.1% and 0.3% (w/w) HPMC, between 0.1% and 1.8% (w/w) PEG-400, and the balance water;
(iii) between 18% and 40% (w/w) ethylene oxide/propylene oxide block copolymer, between 0.05% and 0.8% (w/w) carboxymethylcellulose (CMC), between 0.1% and 2.5% (w/w) PEG-400, and the balance water; and,
(iv) between 12-30% Pluronic F127, between 5-30% Pluronic F68, between 0.05% and 2% (w/w) CMC, between 0.1% and 2.5% (w/w) PEG-400, and the balance water.

10. The composition for use according to any one of claims 1 - 9, wherein said therapeutic agent for treatment of the urinary tract or mucosal or serous membrane is selected from the group comprising Mitomycin C, BCG (bacillus Calmette-Guerin) Deoxrubicin, Valrubicin, and Gemcitabine, Thiotepa, Ethoglucid (Epodyl), Epirubicin, Pirarubicin, Apaziquone,Vicinium and botulinium toxin, and interleukin-2.

11. A pharmaceutical composition for use according to claim 1, further comprising a tight junction modifier / cell membrane permeability enhancer, **characterized in that** at least one of the following is true:
• said composition releases said therapeutic agent over a temperature range of 36 °C - 42 °C and a pH range of between 5.5 and 8.0, at a rate of 80% in a time range of between 3 and 30 hours;
• said composition releases said therapeutic agent over a temperature range of 36 °C - 42 °C and a pH range of between 1 and 9.0, at a rate of 80% in a time range of between 3 and 30 hours; and,
• said composition releases said therapeutic agent over a temperature range of 36 °C - 42 °C and a pH range of between 1 and 9.0, at a rate of 80% in a time range of between 2 and 4 weeks.

12. A pharmaceutical composition for use according to claim 1 or 10 wherein the thermoreversible hydrogel comprises polymers and copolymers chosen from among the following: Polycarboxylic acids such as polyglycolic acid polylactic acid and polyacrylic acid; polyurethanes; polyesters such as poly(ethylene terephthalate); polyamides such as nylon; polyacrylonitriles; polyphosphazenes; polylactones such as polycaprolactone; polyanhydrides such as poly[bis(p-carboxyphenoxy)propane anhydride; polyethylene; polyvinyl chloride; ethylene vinyl acetate; Poloxamer block copolymers of the type PEGPPG-PEG; polyethylene oxide (polyethylene glycol); polypropyleneoxide (polypropylene glycol); polymethylacrylate; polysaccharides as dextrin, cyclodextrins, hydroxypropylmethylcellulose (HPMC), hydroxyethylcellulose, hydroxymethylcellulose, chitosan, polyvinyl alcohol and polyvinyl acetate; Polylactide (PLA) and Poly(Lactide-co- Glycolide) (PLGA); polymer microspheres; starch and modified starches; and cellulose polymers.

13. A pharmaceutical composition for use according to claim 10 or 11 , wherein said thermoreversible hydrogel comprises a composition selected from the group comprising of:
(i) between 20% and 30% (w/w) ethylene oxide/propylene oxide block copolymer, between 0.05% and 0.5% (w/w) hydroxypropylmethylcellulose (HPMC), between 0.1% and 2.5% (w/w) polyethylene glycol (PEG)-400, and the balance water;
(ii) between 20% and 30% (w/w) ethylene oxide/propylene oxide block copolymer, between 0.1% and 0.3% (w/w) HPMC, between 0.1% and 1.8% (w/w) PEG-400, and the balance water;
(iii) between 18% and 40% (w/w) ethylene oxide/propylene oxide block copolymer, between 0.05% and 0.8% (w/w) carboxymethylcellulose (CMC), between 0.1% and 2.5% (w/w) PEG-400, and the balance water; and,
(iv) between 12-30% Pluronic F127, between 5-30% Pluronic F68, between 0.05% and 2% (w/w) CMC, between 0.1% and 2.5% (w/w) PEG-400, and the balance water.

## Patentansprüche

1. Thermoreversible Hydrogelzusammensetzung, umfassend mindestens ein therapeutisches Mittel, ausgewählt aus der Gruppe, bestehend aus antineoplastischen Mitteln, chemotherapeutischen Mitteln, antiinfektiösen Mitteln, antimikrobiellen Mitteln, antiparasitären Mitteln, antiviralen Mitteln, auf das Blut einwirkenden Mitteln, antihämorrhagischen Mitteln, antithrombotischen Mitteln, antimykotischen Antiseptika, Mitteln zur Behandlung von Erkrankungen des Urogenitaltraktes, entzündungshemmenden Mitteln, neurologischen Mitteln, Gentherapeutika, Corticosteroiden, Analgetika und Anästhetika, Wachstumsfaktoren, VEGF, inhibitorischen Faktoren, LIF, Proteinen, Mucin, den Zellzyklus modifizierenden Mitteln zur Synchronisation des Zellzyklus, ausgewählt aus der Gruppe, umfassend Chalone, Colcemid, Methotrexat und Thymidin, und jede Kombination davon, zur Verwendung bei der Behandlung der Harnwege, wobei das therapeutische Mittel in den Harntrakt abgegeben wird; **dadurch gekennzeichnet, dass** die thermoreversible Hydrogelzusammensetzung ein thermoreversibles Hydrogel umfasst, **gekennzeichnet durch**:
eine Viskosität von weniger als 5 Pa·s über einen Temperaturbereich von 4 °C bis 12 °C;
eine Viskosität von mehr als 10³ Pa·s bei 37 °C;
ein "Schälfestigkeits"-Haftvermögen gemäß ASTM-Standard D2256-03 von 0,5 - 5,0 N cm⁻² bei 37 °C; und
ausreichende Flexibilität, sodass ein 3 cm² x 3 cm² großer Abschnitt des Blasengewebes, der mit dem thermoreversiblen Hydrogel bei Raumtemperatur beschichtet wird, ohne Ablösung des thermoreversiblen Hydrogels von dem Blasengewebe auf 9 cm² x 9 cm² gedehnt werden kann, und
wobei das thermoreversible Hydrogel eine Zusammensetzung umfasst, die ein Poloxamer umfasst.

2. Thermoreversible Hydrogelzusammensetzung, umfassend mindestens ein therapeutisches Mittel, ausgewählt aus der Gruppe, bestehend aus antineoplastischen Mitteln, chemotherapeutischen Mitteln, antiinfektiösen Mitteln, antimikrobiellen Mitteln, antiparasitären Mitteln, antiviralen Mitteln, auf das Blut einwirkenden Mitteln, antihämorrhagischen Mitteln, antithrombotischen Mitteln, antimykotischen Antiseptika, Mitteln zur Behandlung von Erkrankungen des Urogenitaltraktes, entzündungshemmenden Mitteln, neurologischen Mitteln, Gentherapeutika, Corticosteroiden, Analgetika und Anästhetika, Wachstumsfaktoren, VEGF, inhibitorischen Faktoren, LIF, Proteinen, Mucin, den Zellzyklus modifizierenden Mitteln zur Synchronisation eines Zellzyklus, ausgewählt aus der Gruppe, umfassend Chalone, Colcemid, Methotrexat und Thymidin, und jede Kombination davon, zur Verwendung bei der Behandlung einer Schleimhaut oder einer serösen Haut, **dadurch gekennzeichnet, dass** die thermoreversible Hydrogelzusammensetzung ein thermoreversibles Hydrogel umfasst, **gekennzeichnet durch**:
eine Viskosität von weniger als 5 Pa·s über einen Temperaturbereich von 4 °C bis 12 °C;
eine Viskosität von mehr als 10³ Pa·s bei 37 °C;
ein "Schälfestigkeits"-Haftvermögen gemäß ASTM-Standard D2256-03 von 0,5 - 5,0 N cm⁻² bei 37 °C; und
ausreichende Flexibilität, sodass ein 3 cm² x 3 cm² großer Abschnitt des Blasengewebes, der mit dem thermoreversiblen Hydrogel bei Raumtemperatur beschichtet wird, ohne Ablösung des thermoreversiblen Hydrogels von dem Blasengewebe auf 9 cm² x 9 cm² gedehnt werden kann, und
wobei das thermoreversible Hydrogel eine Zusammensetzung umfasst, die ein Poloxamer umfasst.

3. Zusammensetzung zur Verwendung nach Anspruch 1 oder 2, wobei das thermoreversible Hydrogel zusätzlich mindestens einen Tight-Junction-Modifikator/Zellmembran-Permeabilitätsverstärker umfasst.

4. Zusammensetzung zur Verwendung nach Anspruch 3, wobei mindestens eine der folgenden Aussagen zutrifft:
der Tight-Junction-Modifikator/Zellmembran-Permeabilitätsverstärker ist ausgewählt aus der Gruppe, umfassend anionische Tenside, nicht-anionische Tenside, geladene Polymere, Dimethylsulfoxid (DMSO), Decylmethylsulfoxid, tert-Butylcyclohexanol, Fettsäuren und deren Ester und Salze, Ethanol, Nicotinamid, Harnstoff, Perfluorpolyether, Monoterpenketone, Dinatriumcitrat, Bernsteinsäure und Tris;
der Tight-Junction-Modifikator/Zellmembran-Permeabilitätsverstärker umfasst ein Tensid, ausgewählt aus der Gruppe, umfassend Polysorbate, Natriumdodecylsulfat und Dextransulfat;
der Tight-Junction-Modifikator/Zellmembran-Permeabilitätsverstärker umfasst ein geladenes Polymer, ausgewählt aus der Gruppe, umfassend Chitosan, Polyarginin, Polylysin und Alginat; und
der Tight-Junction-Modifikator/Zellmembran-Permeabilitätsverstärker umfasst ein Monoterpenketon, ausgewählt aus der Gruppe umfassend, (-)-Menthol, (-)-Menthon, Pfefferminzöl und Spearminzöl.

5. Zusammensetzung zur Verwendung nach einem der Ansprüche 1 bis 4, wobei das thermoreversible Hydrogel zusätzlich mindestens eine Komponente umfasst, ausgewählt aus der Gruppe, umfassend adhäsive und verdickende Verbindungen; Bindemittel; pHmodifizierende Substanzen; Diffusionsbeschichtungen; Weichmacher; Komponenten zur Erhöhung der Permeabilität innerhalb der Rezeptur; quellbare Arzneimittelträger; matrixbildende Polymere; und eine beliebige Kombination davon.

6. Zusammensetzung zur Verwendung nach Anspruch 5, wobei mindestens eine der folgenden Aussagen zutrifft:
die adhäsiven und verdickenden Verbindungen sind ausgewählt aus der Gruppe, umfassend Gummis;
die adhäsiven und verdickenden Verbindungen sind ausgewählt aus der Gruppe, umfassend Guargummi, Johannisbrotkernmehl, Xanthangummi und deren Derivate;
die Bindemittel sind ausgewählt aus der Gruppe umfassend, Polycarbophil, Cellulose, mikrokristalline Cellulose, Cellulosederivate, HPMC, Hydroxypropylcellulose (HPC), niedrig substituierte Hydroxypropylcellulose, Dicalciumphosphat, Lactose, Sucrose, Ethylcellulose, Hydroxypropymethylcellulose-Acetatsuccinat (HPMCAS), Polyvinylpyrrolidon (PVP), Vinylpyrrolidon-/Vinylacetat-Copolymer, Polyethylenglycol, Polyethylenoxid, Polymethacrylate, Polyvinylalkohole (PVA), teilweise hydrolysiertes Polyvinylacetat (PVAc), Polysaccharide, Hyaluronsäure, Fette, Fettsäurederivate und eine beliebige Kombination davon;
die pH-modifizierenden Substanzen sind ausgewählt aus der Gruppe, umfassend Säuren, Basen und Puffer;
die pH-modifizierenden Substanzen sind ausgewählt aus der Gruppe, umfassend Adipinsäure, Äpfelsäure, L-Arginin, Ascorbinsäure, Asparaginsäure, Benzolsulfonsäure, Benzoesäure, Bernsteinsäure, Zitronensäure, Ethansulfonsäure, 2-Hydroxyethansulfonsäure, Fumarsäure, Gluconsäure, Glucuronsäure, Glutaminsäure, Kaliumhydrogentartrat, Maleinsäure, Malonsäure, Methansulfonsäure, Toluolsulfonsäure, Trometamol, Weinsäure, Kaliumhydrogentartrat und ihre jeweiligen Salze, Phosphatsalze und Tris;
die Diffusionsbeschichtungen sind ausgewählt aus der Gruppe, umfassend Ethylcellulosen und Polymethacrylate, Celluloseacetat und Celluloseacetatbutyrat oder eine beliebige Kombination davon;
die Komponenten zur Erhöhung der Permeabilität innerhalb der Rezeptur sind ausgewählt aus der Gruppe, umfassend Polyethylenglycole, PVP, PVA, HPMC, HPC, Hydroxyethylcellulosen (HEC), Methylcellulose (MC), Carboxymethylcellulosen und deren Salze, Dextrine, Maltodextrine, Cylcodextrine, Dextrane, Harnstoff, Salze, Zucker, Zuckeralkohole und eine beliebige Kombination davon;
die quellbaren Hilfsstoffe sind ausgewählt aus der Gruppe, umfassend Polyvinylpyrrolidone, Crospovidone, vernetzte Natriumcarboxymethylcellulose, vernetzte Natriumcarboxymethylstärke, Polyethylenoxide, Polymethacrylate, L-HPC, Celluloseacetat, Ethylcellulose, Polymethacrylate, Polyethylenoxide mit hohem Molekulargewicht, Xanthangummi, Copolymere von Vinylpyrrolidon und Vinylacetat, Polyvinylpyrrolidone, Crospovidone, vernetzte Natriumcarboxymethylcellulose, vernetzte Natriumcarboxymethylstärke, Poly(hydroxyalkylmethacrylat), Alginate, Galactomannane und eine beliebige Kombination davon; und
diese matrixbildenden Polymere sind ausgewählt aus der Gruppe umfassend Hydroxyethylmethylcellulosen, HPC, HEC, MC, Ethylcellulosen, Alkylcellulosen, Hydroxyalkylcellulosen, Hydroxyalkylmethylcellulosen, Natrium-CMCs, Alginate, Galactomannane, Xanthane, Polyethylenoxide, Polyacrylsäuren, Polymethacrylsäuren, Polymethacrylsäurederivate, Polyvinylalkohole, teilweise hydrolysiertes Polyvinylacetat, Polyvinylpyrrolidon, Agar, Pektin, Gummi arabicum, Tragant, Gelatine, Stärke, Stärkederivate, Poly(propylenoxid) (PPO), Poly(lactid-co-glycolsäure) (PLGA), Poly(N-isopropylacrylamid) (PNIPAM), Poly(propylenfumarat) (PPF), Polyurethan (PU), Poly(organophosphazen) (POP), Stearinsäure, Poly(acrylsäure), Glycerylstearat, Cetearylalkohol, Natriumstearoyllactylat, Hydroxylanolin und eine beliebige Kombination davon.

7. Zusammensetzung zur Verwendung nach einem der Ansprüche 1 bis 6, wobei das thermoreversible Hydrogel zusätzlich als biologischer Klebstoff verwendet wird.

8. Zusammensetzung zur Verwendung nach einem der Ansprüche 1 bis 7, wobei das thermoreversible Hydrogel ferner **gekennzeichnet ist durch** mindestens eine Eigenschaft, ausgewählt aus der Gruppe, umfassend:
eine Viskosität von weniger als 0,5 Pa·s über einen Temperaturbereich von 4 °C bis 12 °C;
eine Viskosität von mehr als 3 x 10³ Pa·s bei 37 °C;
ein "Schälfestigkeits"-Haftvermögen gemäß ASTM-Standard D2256-03 von 1,0 - 5,0 N cm⁻² bei 37 °C; und
Flexibilität, die ausreicht, um zu ermöglichen, dass sich das Volumen des Gewebes, auf das die Zusammensetzung aufgetragen wird, um einen Faktor von mindestens 3 ohne Ablösung des Gels von dem Gewebe ausdehnt.

9. Zusammensetzung zur Verwendung nach einem der Ansprüche 1 bis 7, wobei das thermoreversible Hydrogel eine Zusammensetzung umfasst, die ausgewählt ist aus der Gruppe, bestehend aus:
(i) zwischen 20 und 30 Gew.-% Ethylenoxid/Propylenoxid-Blockcopolymer, zwischen 0,05 und 0,5 Gew.-% Hydroxypropylmethylcellulose (HPMC), zwischen 0,1 und 2,5 Gew.-% Polyethylenglycol (PEG)-400 und der Rest Wasser;
(ii) zwischen 20 und 30 Gew.-% Ethylenoxid/Propylenoxid-Blockcopolymer, zwischen 0,1 und 0,3 Gew.-% HPMC, zwischen 0,1 und 1,8 Gew.-% PEG-400 und der Rest Wasser;
(iii) zwischen 18 und 40 Gew.-% Ethylenoxid/Propylenoxid-Blockcopolymer, zwischen 0,05 und 0,8 Gew.-% Carboxymethylcellulose (CMC), zwischen 0,1 und 2,5 Gew.-% PEG-400 und der Rest Wasser; und
(iv) zwischen 12 und 30 % Pluronic F127, zwischen 5-30 % Pluronic F68, zwischen 0,05 Gew.-% und 2 Gew.-% CMC, zwischen 0,1 Gew.-% und 2,5 Gew.-% PEG-400 und der Rest Wasser.

10. Zusammensetzung zur Verwendung nach einem der Ansprüche 1 bis 9, wobei das therapeutische Mittel zur Behandlung der Harnwege oder der Schleimhaut oder der serösen Haut aus der Gruppe ausgewählt ist, umfassend Mitomycin C, BCG (Bacillus Calmette-Guerin), Deoxrubicin, Valrubicin und Gemcitabin, Thiotepa, Ethoglucid (Epodyl), Epirubicin, Pirarubicin, Apaziquon, Vicinium und Botulinumtoxin und Interleukin-2.

11. Pharmazeutische Zusammensetzung zur Verwendung nach Anspruch 1, ferner umfassend einen Tight-Junction-Modifikator/Zellmembran-Permeabilitätsverstärker, **dadurch gekennzeichnet, dass** mindestens eine der folgenden Aussagen zutrifft:
• die Zusammensetzung setzt das therapeutische Mittel über einen Temperaturbereich von 36 °C bis 42 °C und einen pH-Bereich von zwischen 5,5 und 8,0 mit einer Rate von 80 % in einem Zeitbereich von zwischen 3 und 30 Stunden frei;
• die Zusammensetzung setzt das therapeutische Mittel über einen Temperaturbereich von 36 °C bis 42 °C und einen pH-Bereich von zwischen 1 und 9,0 mit einer Rate von 80 % in einem Zeitbereich von zwischen 3 und 30 Stunden frei; und
• die Zusammensetzung setzt das therapeutische Mittel über einen Temperaturbereich von 36 °C bis 42 °C und einen pH-Bereich von zwischen 1 und 9,0 mit einer Rate von 80 % in einem Zeitbereich von zwischen 2 und 4 Wochen frei.

12. Pharmazeutische Zusammensetzung zur Verwendung nach Anspruch 1 oder 10, wobei das thermoreversible Hydrogel Polymere und Copolymere umfasst, die aus den folgenden ausgewählt sind: Polycarbonsäuren, wie beispielsweise Polyglycolsäure, Polymilchsäure und Polyacrylsäure; Polyurethanen; Polyestern, wie beispielsweise Poly(ethylenterephthalat); Polyamiden, wie Nylon; Polyacrylnitrile; Polyphosphazene; Polylactone wie Polycaprolacton; Polyanhydride wie Poly[bis (p-carboxyphenoxy) propananhydrid; Polyethylen; Polyvinylchlorid; Ethylenvinylacetat; Poloxamer-Blockcopolymere vom Typ PEGPPG-PEG; Polyethylenoxid (Polyethylenglycol); Polypropylenoxid (Polypropylenglyol); Polymethylacrylat; Polysacchariden, wie beispielsweise Dextrin, Cyclodextrine, Hydroxypropylmethylcellulose (HPMC), Hydroxyethylcellulose, Hydroxymethylcellulose, Chitosan, Polyvinylalkohol und Polyvinylacetat; Polylactid (PLA) und Poly(Lactid-co-Glycolid) (PLGA); Polymermikrokugeln; Stärke und modifizierten Stärken; und Cellulosepolymeren.

13. Pharmazeutische Zusammensetzung zur Verwendung nach Anspruch 10 oder 11, wobei das thermoreversible Hydrogel eine Zusammensetzung umfasst, ausgewählt aus der Gruppe bestehend aus:
(i) zwischen 20 und 30 Gew.-% Ethylenoxid/Propylenoxid-Blockcopolymer, zwischen 0,05 und 0,5 Gew.-% Hydroxypropylmethylcellulose (HPMC), zwischen 0,1 und 2,5 Gew.-% Polyethylenglycol(PEG)-400 und der Rest Wasser;
(ii) zwischen 20 und 30 Gew.-% Ethylenoxid/Propylenoxid-Blockcopolymer, zwischen 0,1 und 0,3 Gew.-% HPMC, zwischen 0,1 und 1,8 Gew.-% PEG-400 und der Rest Wasser;
(iii) zwischen 18 und 40 Gew.-% Ethylenoxid/Propylenoxid-Blockcopolymer, zwischen 0,05 und 0,8 Gew.-% Carboxymethylcellulose (CMC), zwischen 0,1 und 2,5 Gew.-% PEG-400 und der Rest Wasser; und
(iv) zwischen 12-30 % Pluronic F127, zwischen 5-30 % Pluronic F68, zwischen 0,05 Gew.-% und 2 Gew.-% CMC, zwischen 0,1 Gew.-% und 2,5 Gew.-% PEG-400 und der Rest Wasser.

## Revendications

1. Composition d'hydrogel thermoréversible comprenant au moins un agent thérapeutique choisi dans le groupe constitué d'agents antinéoplasiques, agents chimiothérapeutiques, agents anti-infectieux, agents antimicrobiens, agents antiparasitaires, agents antiviraux, agents agissant sur le sang, antihémorragiques, agents antithrombotiques, antiseptiques antifongiques, agents pour le traitement des maladies du système génito-urinaire, agents anti-inflammatoires, agents neurologiques, agents de thérapie génique, corticostéroïdes, agents analgésiques et anesthésiques, facteurs de croissance, facteur de croissance endothélial vasculaire (FCEV), facteurs inhibiteurs, facteur inhibiteur de la leucémie (LIF), protéines, mucine, agents modificateurs du cycle cellulaire pour la synchronisation du cycle cellulaire choisis dans le groupe comprenant les chalones, le colcémide, le méthotrexate et la thymidine, et n'importe quelle combinaison de ceux-ci, pour une utilisation dans le traitement des voies urinaires, dans laquelle l'agent thérapeutique est administré à l'appareil urinaire ; **caractérisée en ce que** ladite composition d'hydrogel thermoréversible comprend un hydrogel thermoréversible **caractérisé par** :
une viscosité inférieure à 5 Pa·s sur une plage de température de 4 °C à 12 °C ;
une viscosité supérieure à 10³ Pa·s à 37 °C ;
une adhésivité de « résistance au pelage » selon la norme ASTM D2256-03 de 0,5 à 5,0 N cm⁻² à 37 °C ; et,
une flexibilité suffisante de telle sorte qu'une section de 3 cm² x 3 cm² de tissu de vessie en couches avec ledit hydrogel thermoréversible à la température ambiante peut être étirée à 9 cm² x 9 cm² sans détachement dudit hydrogel thermoréversible par rapport audit tissu de vessie, et
dans laquelle ledit hydrogel thermoréversible comprend une composition comprenant un poloxamère.

2. Composition d'hydrogel thermoréversible comprenant au moins un agent thérapeutique choisi dans le groupe constitué d'agents antinéoplasiques, agents chimiothérapeutiques, agents anti-infectieux, agents antimicrobiens, agents antiparasitaires, agents antiviraux, agents agissant sur le sang, antihémorragiques, agents antithrombotiques, antiseptiques antifongiques, agents pour le traitement des maladies du système génito-urinaire, agents anti-inflammatoires, agents neurologiques, agents de thérapie génique, corticostéroïdes, agents analgésiques et anesthésiques, facteurs de croissance, facteur de croissance endothélial vasculaire (FCEV), facteurs inhibiteurs, facteur inhibiteur de la leucémie (LIF), protéines, mucine, agents modificateurs du cycle cellulaire pour la synchronisation du cycle cellulaire choisis dans le groupe comprenant les chalones, le colcémide, le méthotrexate et la thymidine, et n'importe quelle combinaison de ceux-ci, pour une utilisation dans le traitement d'une membrane muqueuse ou séreuse, **caractérisée en ce que** ladite composition d'hydrogel thermoréversible comprend un hydrogel thermoréversible **caractérisé par** :
une viscosité inférieure à 5 Pa·s sur une plage de température de 4 °C à 12 °C ;
une viscosité supérieure à 10³ Pa·s à 37 °C ;
une adhésivité de « résistance au pelage » selon la norme ASTM D2256-03 de 0,5 à 5,0 N cm⁻² à 37 °C ; et,
une flexibilité suffisante de telle sorte qu'une section de 3 cm² x 3 cm² de tissu de vessie en couches avec ledit hydrogel thermoréversible à la température ambiante peut être étirée à 9 cm² x 9 cm² sans détachement dudit hydrogel thermoréversible par rapport audit tissu de vessie, et
dans laquelle ledit hydrogel thermoréversible comprend une composition comprenant un poloxamère.

3. Composition pour utilisation selon la revendication 1 ou 2, dans laquelle ledit hydrogel thermoréversible comprend en outre au moins un modificateur des jonctions serrées / amplificateur de perméabilité de membrane cellulaire.

4. Composition pour utilisation selon la revendication 3, dans laquelle au moins l'une des affirmations suivantes est vraie :
ledit modificateur des jonctions serrées / amplificateur de perméabilité de membrane cellulaire est choisi dans le groupe comprenant des agents tensioactifs anioniques, des agents tensioactifs non anioniques, des polymères chargés, du sulfoxyde de diméthyle (DMSO), du sulfoxyde de décylméthyle, du tert-butyl cyclohexanol, des acides gras, leurs esters et sels, de l'éthanol, du nicotinamide, de l'urée, du perfluoropolyéther, des cétones monoterpéniques, du citrate disodique, l'acide succinique et le tris ;
ledit modificateur des jonctions serrées / amplificateur de perméabilité de membrane cellulaire comprend un agent tensioactif choisi dans le groupe comprenant des polysorbates, du dodécylsulfate de sodium et du sulfate de dextrane ;
ledit modificateur des jonctions serrées / amplificateur de perméabilité de membrane cellulaire comprend un polymère chargé choisi dans le groupe comprenant le chitosane, la poly-arginine, la polylysine et un alginate ; et,
ledit modificateur des jonctions serrées / amplificateur de perméabilité de membrane cellulaire comprend une cétone monoterpénique choisie dans le groupe comprenant le (-)-menthol, la (-)-menthone, l'essence de menthe poivrée, et l'essence de menthe verte.

5. Composition pour utilisation selon l'une quelconque des revendications 1 à 4, dans laquelle ledit hydrogel thermoréversible comprend en outre au moins un composant choisi dans le groupe comprenant des agents adhésifs et épaississants ; des liants ; des substances modifiant le pH ; des revêtements de diffusion ; des plastifiants ; des composants pour augmenter la perméabilité au sein de la formulation ; des excipients pouvant gonfler ; des polymères de formation de matrice ; et n'importe quelle combinaison de ceux-ci.

6. Composition pour utilisation selon la revendication 5, dans laquelle au moins l'une des affirmations suivantes est vraie :
lesdits agents adhésifs et épaississants sont choisis dans le groupe comprenant des gommes ;
lesdits agents adhésifs et épaississants sont choisis dans le groupe comprenant la gomme de guar, la gomme de caroube, la gomme de xanthane et leurs dérivés ;
lesdits liants sont choisis dans le groupe comprenant un polycarbophile, de la cellulose, de la cellulose microcristalline, des dérivés de cellulose, de la HPMC, de l'hydroxypropylcellulose (HPC), de l'hydroxypropylcellulose faiblement substituée, du phosphate dicalcique, du lactose, du saccharose, de l'éthylcellulose, de l'acétate-succinate d'hydroxypropyméthylcellulose (HPMCAS), de la polyvinylpyrrolidone (PVP), un copolymère vinylpyrrolidone/acétate de vinyle, du polyéthylène glycol, de l'oxyde de polyéthylène, des polyméthacrylates, des alcools polyvinyliques (PVA), de l'acétate de polyvinyle partiellement hydrolysé (PVAc), des polysaccharides, de l'acide hyaluronique, des matières grasses, des dérivés d'acide gras, et n'importe quelle combinaison de ceux-ci ;
lesdites substances modifiant le pH sont choisies dans le groupe comprenant des acides, des bases et des tampons ;
lesdites substances modifiant le pH sont choisies dans le groupe comprenant l'acide adipique, l'acide malique, la L-arginine, l'acide ascorbique, l'acide aspartique, l'acide benzène-sulfonique, l'acide benzoïque, l'acide succinique, l'acide citrique, l'acide éthanesulfonique, l'acide 2-hydroxyéthanesulfonique, l'acide fumarique, l'acide gluconique, l'acide glucuronique, l'acide glutamique, l'hydrogénotartrate de potassium, l'acide maléique, l'acide malonique, l'acide méthanesulfonique, l'acide toluènesulfonique, le trométamol, l'acide tartrique, l'hydrogénotartrate de potassium et leurs sels respectifs, des sels de phosphate, et le Tris ;
lesdits revêtements de diffusion sont choisis dans le groupe comprenant des éthylcelluloses et polyméthacrylates, de l'acétate de cellulose et de l'acétate-butyrate de cellulose ou n'importe quelle combinaison de ceux-ci ;
lesdits composants pour augmenter la perméabilité au sein de la formulation sont choisis dans le groupe comprenant des polyéthylène glycols, du PVP, du PVA, de la HPMC, de la HPC, des hydroxyéthylcelluloses (HEC), de la méthylcellulose (MC), des carboxyméthylcelluloses et leurs sels, des dextrines, des maltodextrines, des cyclodextrines, des dextranes, de l'urée, des sels, des sucres, des alcools de sucre, et n'importe quelle combinaison de ceux-ci ;
lesdits excipients pouvant gonfler sont choisis dans le groupe comprenant des polyvinylpyrrolidones, des crospovidones, de la carboxyméthylcellulose de sodium réticulée, du carboxyméthylamidon de sodium réticulé, des oxydes de polyéthylène, des polyméthylacrylates, de la L-HPC, de l'acétate de cellulose, de l'éthylcellulose, des polyméthacrylates, des oxydes de polyéthylène à masse moléculaire élevée, de la gomme de xanthane, des copolymères de vinylpyrrolidone et d'acétate de vinyle, des polyvinylpyrrolidones, des crospovidones, de la carboxyméthylcellulose de sodium réticulée, du carboxyméthylamidon de sodium réticulé, poly(méthacrylate d'hydroxyalkyle), des alginates, des galactomannanes, et n'importe quelle combinaison de ceux-ci ; et,
lesdits polymères de formation de matrice sont choisis dans le groupe comprenant des hydroxyéthylméthylcelluloses, de la HPC, de la HEC, de la MC, des éthylcelluloses, des alkylcelluloses, des hydroxyalkylcelluloses, des hydroxyalkylméthylcelluloses, des carboxyméthylcelluloses de sodium, des alginates, des galactomannanes, des xanthanes, des oxydes de polyéthylène, des acides polyacryliques, des acides polyméthacryliques, des dérivés d'acide polyméthacrylique, des alcools polyvinyliques, de l'acétate de polyvinyle partiellement hydrolysé, de la polyvinylpyrrolidone, de l'agar-agar, de la pectine, de la gomme arabique, du tragacanthe, de la gélatine, de l'amidon, des dérivés d'amidon poly(oxyde de propylène) (PPO), du poly(lactide-co-acide glycolique) (PLGA), du poly(N-isopropylacrylamide) (PNIPAM), du poly(fumarate de propylène) (PPF), du polyuréthane (PU), du poly(organophosphazène) (POP), de l'acide stéarique, du poly(acide acrylique), du stéarate de glycéryle, de l'alcool cétéarylique, du stéaroyl-lactylate de sodium, de l'hydroxy-lanoline, et n'importe quelle combinaison de ceux-ci.

7. Composition pour utilisation selon l'une quelconque des revendications 1 à 6, dans laquelle ledit hydrogel thermoréversible est de plus utilisé en tant que colle biologique.

8. Composition pour utilisation selon l'une quelconque des revendications 1 à 7, dans laquelle ledit hydrogel thermoréversible est **caractérisé en outre par** au moins une caractéristique choisie dans le groupe comprenant :
une viscosité inférieure à 0,5 Pa·s sur une plage de température de 4 °C à 12 °C ;
une viscosité supérieure à 3 x 10³ Pa·s à 37 °C ;
une adhésivité de « résistance au pelage » selon la norme ASTM D2256-03 de 1,0 à 5,0 N cm⁻² à 37 °C ; et,
une flexibilité suffisante pour permettre au volume du tissu sur lequel est appliquée ladite composition de s'accroître d'un facteur d'au moins 3 sans détachement dudit gel par rapport audit tissu.

9. Composition pour utilisation selon l'une quelconque des revendications 1 à 7, dans laquelle ledit hydrogel thermoréversible comprend une composition choisie dans le groupe comprenant :
(i) entre 20 % et 30 % (en poids) de copolymère séquencé oxyde d'éthylène/oxyde de propylène, entre 0,05 % et 0,5 % (en poids) d'hydroxypropylméthylcellulose (HPMC), entre 0,1 % et 2,5 % (en poids) de polyéthylène glycol (PEG)-400, et le complément d'eau ;
(ii) entre 20 % et 30 % (en poids) de copolymère séquencé oxyde d'éthylène/oxyde de propylène, entre 0,1 % et 0,3 % (en poids) de HPMC, entre 0,1 % et 1,8 % (en poids) de PEG-400, et le complément d'eau ;
(iii) entre 18 % et 40 % (en poids) de copolymère séquencé oxyde d'éthylène/oxyde de propylène, entre 0,05 % et 0,8 % (en poids) de carboxyméthylcellulose (CMC), entre 0,1 % et 2,5 % (en poids) de PEG-400, et le complément d'eau ; et,
(iv) entre 12 et 30 % de Pluronic F127, entre 5 et 30 % de Pluronic F68, entre 0,05 % et 2 % (en poids) de CMC, entre 0,1 % et 2,5 % (en poids) de PEG-400, et le complément d'eau.

10. Composition pour utilisation selon l'une quelconque des revendications 1 à 9, dans laquelle ledit agent thérapeutique pour traitement de l'appareil urinaire ou de la membrane muqueuse ou séreuse est choisi dans le groupe comprenant Mitomycine C, BCG (bacille Calmette-Guerin) Deoxrubicin, Valrubicine et Gemcitabine, Thiotépa, Éthoglucide (Epodyl), Épirubicine, Pirarubicine, Apaziquone, Vicinium et la toxine botulique, et l'interleukine-2.

11. Composition pharmaceutique pour utilisation selon la revendication 1, comprenant en outre un modificateur des jonctions serrées / amplificateur de perméabilité de membrane cellulaire, **caractérisé en ce qu'**au moins une des affirmations suivantes est vraie :
• ladite composition libère ledit agent thérapeutique sur une plage de température de 36 °C à 42 °C et une plage de pH comprise entre 5,5 et 8,0, à un taux de 80 % dans une plage de temps comprise entre 3 et 30 heures ;
• ladite composition libère ledit agent thérapeutique sur une plage de température de 36 °C à 42 °C et une plage de pH comprise entre 1 et 9,0, à un taux de 80 % dans une plage de temps comprise entre 3 et 30 heures ; et,
• ladite composition libère ledit agent thérapeutique sur une plage de température de 36 °C à 42 °C et une plage de pH comprise entre 1 et 9,0, à un taux de 80 % dans une plage de temps comprise entre 2 et 4 semaines.

12. Composition pharmaceutique pour utilisation selon la revendication 1 ou 10, dans laquelle l'hydrogel thermoréversible comprend des polymères et copolymères choisis parmi les substances suivantes : des acides polycarboxyliques tels que l'acide polyglycolique, l'acide polylactique et l'acide polyacrylique ; des polyuréthanes ; des polyesters tels que le poly(éthylène téréphtalate) ; des polyamides tels que le nylon ; des polyacrylonitriles ; des polyphosphazènes ; des polylactones telles que la polycaprolactone ; des polyanhydrides tels que l'anhydride de poly[bis(p-carboxyphénoxy)propane ; le polyéthylène ; le chlorure de polyvinyle ; l'éthylène-acétate de vinyle ; des copolymères séquencés de poloxamère du type PEGPPG-PEG ; l'oxyde de polyéthylène (polyéthylène glycol) ; l'oxyde de polypropylène (polypropylène glycol) ; un polyméthylacrylate ; des polysaccharides tels que la dextrine, des cyclodextrines, de l'hydroxypropylméthylcellulose (HPMC), de l'hydroxyéthylcellulose, de l'hydroxyméthylcellulose, du chitosane, de l'alcool polyvinylique et de l'acétate de polyvinyle ; du polylactide (PLA) et du poly(lactide-co-glycolide) (PLGA) ; des microsphères de polymère ; de l'amidon et des amidons modifiés ; et des polymères de cellulose.

13. Composition pharmaceutique pour utilisation selon la revendication 10 ou 11, dans laquelle ledit hydrogel thermoréversible comprend une composition choisie dans le groupe comprenant :
(i) entre 20 % et 30 % (en poids) de copolymère séquencé oxyde d'éthylène/oxyde de propylène, entre 0,05 % et 0,5 % (en poids) d'hydroxypropylméthylcellulose (HPMC), entre 0,1 % et 2,5 % (en poids) de polyéthylène glycol (PEG)-400, et le complément d'eau ;
(ii) entre 20 % et 30 % (en poids) de copolymère séquencé oxyde d'éthylène/oxyde de propylène, entre 0,1 % et 0,3 % (en poids) de HPMC, entre 0,1 % et 1,8 % (en poids) de PEG-400, et le complément d'eau ;
(iii) entre 18 % et 40 % (en poids) de copolymère séquencé oxyde d'éthylène/oxyde de propylène, entre 0,05 % et 0,8 % (en poids) de carboxyméthylcellulose (CMC), entre 0,1 % et 2,5 % (en poids) de PEG-400, et le complément d'eau ; et,
(iv) entre 12 et 30 % de Pluronic F127, entre 5 et 30 % de Pluronic F68, entre 0,05 % et 2 % (en poids) de CMC, entre 0,1 % et 2,5 % (en poids) de PEG-400, et le complément d'eau.
